# EUROPEAN PATENT APPLICATION

(11) **EP 4 450 488 A1**
(43) Date of publication of application: **23.10.2024**
(21) Application number: 22906676.6
(22) Date of filing: 16.12.2022
(51) Int. Cl.: C07D 209/18, A61K 31/4025, A61P 17/06, A61P 31/00

(54) **CRYSTALLINE FORM OF AROMATIC RING DERIVATIVE, AND PREPARATION METHOD THEREFOR AND APPLICATION THEREOF**

(30) Priority: 17.12.2021 CN 202111555458
(71) Applicant: Shanghai Jemincare Pharmaceutical Co., Ltd., Shanghai 201315 (CN)
(72) Inventor: ZHANG, Yong, Shanghai 201203 (CN); YE, Longbing, Shanghai 201203 (CN); KANG, Zhangping, Shanghai 201203 (CN); CHENG, Hongming, Shanghai 201203 (CN); PENG, Jianbiao, Shanghai 201203 (CN)
(74) Representative: Greaves Brewster LLP
(86) International application number: PCT/CN2022/139461
(87) International publication number: WO 2023/109931

(57) **Abstract**

Disclosed are a crystalline form of an aromatic ring derivative, and a preparation method therefor and an application thereof. Specifically, disclosed are a salt form, a crystalline form, and a pharmaceutical composition of a compound of formula (I), and a use thereof.

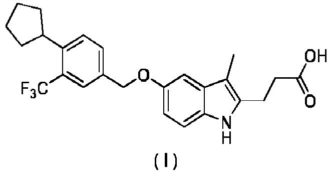

## Description

The present application claims the right of the following priority:
CN 202111555458.X, filed on December 17, 2021.

### TECHNICAL FIELD

The present disclosure relates to a crystal form, a salt form, a pharmaceutical composition of a compound of formula (I), and a use thereof as an SIP1 agonist.

### BACKGROUND

Sphingosine-1-phosphate (S1P) is an amphoteric biological signal molecule belonging to lysophospholipid (LP). S1P can activate complex downstream signals by acting on five G protein-coupled receptor subtypes-sphingosine-1-phosphate receptor (S1PR1-5), thereby regulating important physiological and biochemical functions. S1P binds to different S1P receptors to regulate different physiological functions and plays an important role in maintaining the health of the body and in the occurrence of diseases.

S1P1 receptor agonists interfere with lymphocyte trafficking, sequestering them in lymph nodes and other secondary lymphoid tissues. This leads to a decrease in peripheral circulating lymphocytes, and the clinical value of lymphocyte isolation is to exclude them from the visual field of inflammation and/or autoimmune response in surrounding tissues. This isolation of lymphocytes (e.g., in lymph nodes) is considered to be the result of the following simultaneous actions: agonist-driven functional antagonism of S1P1 receptors on T cells (thus reducing the ability of S1P to mobilize T cells out of lymph nodes) and continuous agonism of S1P1 receptors on lymph node endothelium (thus enhancing barrier function against lymphocyte migration). Therefore, S1P1 receptor agonists can reduce human autoimmune ability by preventing lymphocyte transportation, so they can be used as immunosuppressants to treat various autoimmune diseases.

Among them, the S1P1 agonist Fingolimod (FTY720) was approved by the FDA for the treatment of Multiple Scleorosis (MS), which had developed a new therapeutic field for the treatment of immune diseases. Although FTY720 has clinical efficacy, it is a non-selective S1P receptor agonist. The combination of FTY720 with S1P3 *in vivo* often leads to a series of important side effects, such as bradycardia, which greatly limits its use in the treatment of immune diseases. Therefore, the discovery of second-generation of highly selective S1P1 agonists with better efficacy, less side effects and wider range of uses in the treatment of immune diseases has become one of the hot topics in drug research.

An S1P1 agonist is provided in the application with the application number PCT/CN2019/123485 (filing date: December 6, 2019), with the structure shown as follows:

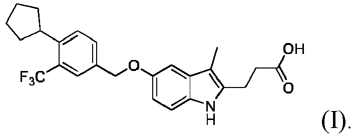

### CONTENT OF THE PRESENT INVENTION

The present disclosure provides a calcium salt of a compound of formula (I),

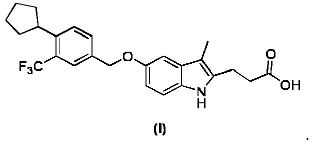

In some embodiments of the present disclosure, the calcium salt is

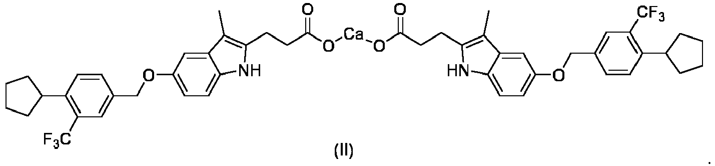

The present disclosure further provides a crystal form G of a calcium salt of a compound of formula (II), wherein the crystal form G has an X-ray powder diffraction pattern comprising characteristic peaks at the following 2Θ angles: 8.98 ± 0.2°, 10.12 ± 0.2°, 13.56 ± 0.2°, 14.66 ± 0.2°, 15.22 ± 0.2°, 18.48 ± 0.2°.

In some embodiments of the present disclosure, the X-ray powder diffraction pattern of the crystal form G comprises characteristic peaks at the following 2Θ angles: 8.98 ± 0.2°, 10.12 ± 0.2°, 13.56 ± 0.2°, 14.66 ± 0.2°, 15.22 ± 0.2°, 16.34 ± 0.2°, 16.92 ± 0.2°, 18.48 ± 0.2°, 22.67 ± 0.2°, 23.06 ± 0.2°.

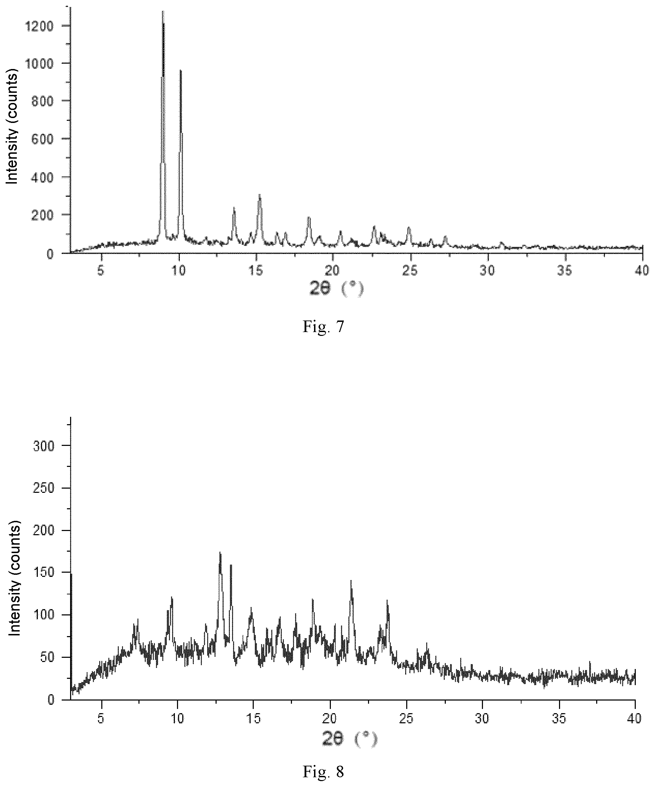

In some embodiments of the present disclosure, the X-ray powder diffraction pattern of the crystal form G is an X-ray powder diffraction pattern basically as shown in Fig. 7.

In some embodiments of the present disclosure, analytical data for the X-ray powder diffraction pattern of the crystal form G is as shown in Table 7.

**Table 7**

| Pos. [°2Th.] | Height [cts] | FWHM Left [°2Th.] | d-spacing [Å] | Rel. Int. [%] |
|---|---|---|---|---|
| 8.98 | 1219.24 | 0.12 | 9.84 | 100.00 |
| 10.12 | 903.05 | 0.14 | 8.74 | 74.07 |
| 13.56 | 187.67 | 0.14 | 6.53 | 15.39 |
| 14.66 | 63.10 | 0.12 | 6.04 | 5.18 |
| 15.22 | 265.12 | 0.10 | 5.82 | 21.74 |
| 16.34 | 70.68 | 0.16 | 5.42 | 5.80 |
| 16.92 | 70.96 | 0.14 | 5.24 | 5.82 |
| 18.48 | 144.72 | 0.14 | 4.80 | 11.87 |
| 19.13 | 47.76 | 0.24 | 4.64 | 3.92 |
| 20.44 | 75.91 | 0.16 | 4.34 | 6.23 |
| 21.18 | 28.48 | 0.39 | 4.19 | 2.34 |
| 22.67 | 89.34 | 0.18 | 3.92 | 7.33 |
| 23.06 | 68.88 | 0.14 | 3.86 | 5.65 |
| 24.90 | 99.38 | 0.16 | 3.58 | 8.15 |
| 26.32 | 37.92 | 0.16 | 3.39 | 3.11 |
| 27.24 | 56.07 | 0.16 | 3.27 | 4.60 |
| 30.88 | 27.23 | 0.24 | 2.90 | 2.23 |

In some embodiments of the present disclosure, the crystal form G further comprises water and a solvent;
wherein
the solvent is selected from ethanol and isopropanol;
the content of the solvent ranges from 0.1% to 6.0%;
the content of water ranges from 0.1 to 4.0%.

In some embodiments of the present disclosure, the content of the solvent ranges from 1.0% to 5.5%, and the content of water ranges from 3.0% to 4.5%.

In some embodiments of the present disclosure, the solvent is ethanol, the content of ethanol ranges from 1.0% to 5.5%, and the content of water ranges from 3.0% to 4.5%.

In some embodiments of the present disclosure, the content of the solvent ranges from 1.64% to 4.77%, and the content of water ranges from 3.09% to 4.37%.

In some embodiments of the present disclosure, the solvent is ethanol, the content of ethanol ranges from 1.64% to 4.77%, and the content of water ranges from 3.09% to 4.37%.

In some embodiments of the present disclosure, the solvent is ethanol, wherein the content of ethanol is 4.77%, and the content of water is 3.09%.

In some embodiments of the present disclosure, the solvent is ethanol, wherein the content of ethanol is 3.05%, and the content of water is 3.89%.

In some embodiments of the present disclosure, the solvent is ethanol, wherein the content of ethanol is 1.97%, and the content of water is 4.37%.

In some embodiments of the present disclosure, the solvent is ethanol, wherein the content of ethanol is 1.64%, and the content of water is 3.75%.

The characteristic diffraction peaks used in this specification are selected from peaks in the observed diffraction patterns. When distinguishing a plurality of crystalline solids, compared to the size of the peak, a peak that is visible in the crystalline solid but not visible in other crystalline solids becomes a preferred characteristic peak for identifying the crystalline solid. Even one or two such characteristic peaks can characterize the crystalline solid.

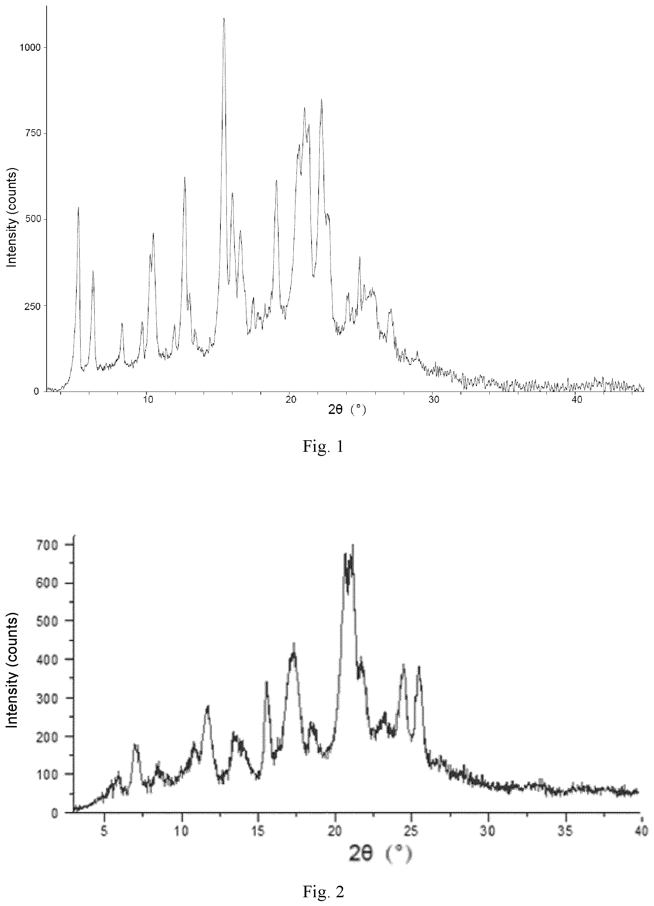

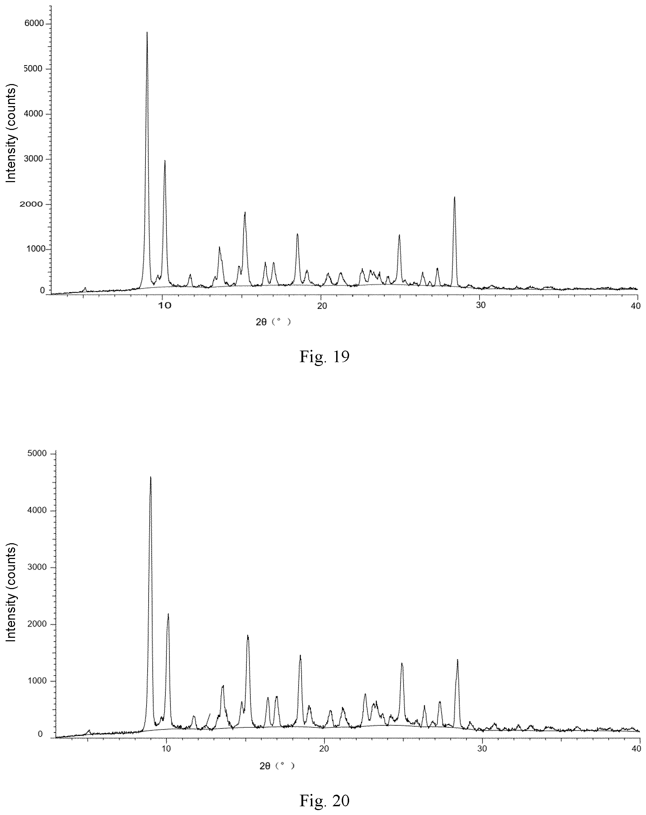

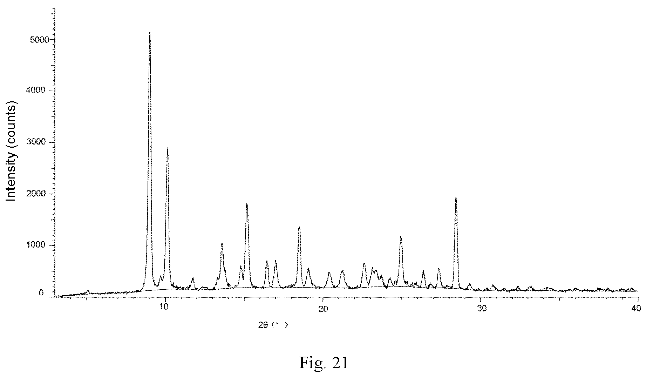

Particularly, the crystal form G can be distinguished from other crystal forms (e.g., anhydrous substance, etc.) disclosed in this specification by the presence of characteristic diffraction peaks. Additionally, comparing the measured charts, if their characteristic peaks are consistent, the powder X-ray diffraction patterns can be considered to be substantially identical. The crystal form G can exhibit changes in solvent hydration state due to changes in solvent and water content. The crystal form G with different solvent hydration contents has common characteristic peaks, as shown in Figs. 1 and 18-21.

In some embodiments of the present disclosure, the common characteristic peaks are at least three peaks selected from diffraction angles (2θ): 8.98 ± 0.2°, 10.12 ± 0.2°, 13.56 ± 0.2°, 14.66 ± 0.2°, 15.22 ± 0.2°, 18.48 ± 0.2°.

In some embodiments of the present disclosure, the common characteristic peaks are at least three peaks selected from the diffraction angles (2θ): 8.98 ± 0.2°, 10.12 ± 0.2°, 13.56 ± 0.2°, 14.66 ± 0.2°, 15.22 ± 0.2°, 16.34 ± 0.2°, 16.92 ± 0.2°, 18.48 ± 0.2°, 22.67 ± 0.2°, and 23.06 ± 0.2°.

It is to be noted that the content (%) in the present disclosure represents the mass ratio, with the unit expressed as g/g.

In yet another aspect of the present disclosure, the present disclosure further provides a method for preparing the crystal form G. According to the embodiments of the present disclosure, the method comprises: dissolving the compound of formula (I) in a sodium ethoxide solution, followed by stirring in the presence of calcium chloride, filtering, and drying under reduced pressure to obtain the crystal form G. This method differs from general methods for forming calcium salt crystal forms in the prior art. Specifically, the method of the present disclosure utilizes conditions with calcium chloride instead of calcium hydroxide, and the crystal form G can only be obtained after drying.

In some embodiments of the present disclosure, the method comprises: heating the compound of formula (I) and anhydrous ethanol to 35-45°C, stirring for 5-30 minutes, adding a sodium ethoxide solution, stirring for 2-3 hours, adding dropwise a calcium chloride solution, stirring and reacting for 1-3 hours, cooling to 20-30°C, stirring for 4-6 hours, filtering, rinsing a filter cake with purified water and ethanol, and drying the filter cake under reduced pressure at 25-35°C to obtain the crystal form G.

In some embodiments of the present disclosure, the mass ratio of sodium ethoxide to anhydrous ethanol in the sodium ethoxide solution is (0.01-0.05):(0.40-0.80).

In some embodiments of the present disclosure, the mass ratio of sodium ethoxide to anhydrous ethanol in the sodium ethoxide solution is 0.035:0.631.

In some embodiments of the present disclosure, the mass ratio of calcium chloride to pure water in the calcium chloride solution is (0.01-0.08):(0.20-1.00).

In some embodiments of the present disclosure, the mass ratio of calcium chloride to pure water in the calcium chloride solution is 0.040:0.847.

The present disclosure further provides a crystal form A of a compound of formula (I), 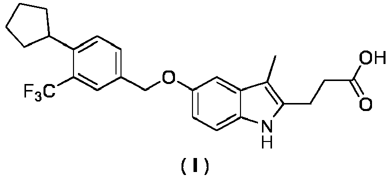 wherein the crystal form A has an X-ray powder diffraction pattern comprising characteristic peaks at the following 2θ angles: 5.25 ± 0.2°, 6.28 ± 0.2°, 10.5 ± 0.2°, 12.69 ± 0.2°, 15.45 ± 0.2°, 16.02 ± 0.2°.

In some embodiments of the present disclosure, the X-ray powder diffraction pattern of the crystal form A comprises characteristic peaks at the following 2θ angles: 5.25 ± 0.2°, 6.28 ± 0.2°, 10.5 ± 0.2°, 12.69 ± 0.2°, 15.45 ± 0.2°, 16.02 ± 0.2°, 16.60 ± 0.2°, 20.66 ± 0.2°, 21.39 ± 0.2°, 22.28 ± 0.2°.

In some embodiments of the present disclosure, the X-ray powder diffraction pattern of the crystal form A is an X-ray powder diffraction pattern basically as shown in Fig. 1.

In some embodiments of the present disclosure, analytical data for the X-ray powder diffraction pattern of the crystal form A is as shown in Table 1.

**Table 1**

| Pos. [°2Th.] | Height [cts] | FWHM Left [°2Th.] | d-spacing [Å] | Rel. Int. [%] |
|---|---|---|---|---|
| 5.25 | 957 | 0.26 | 16.82 | 54.9 |
| 6.28 | 647 | 0.24 | 14.07 | 37.1 |
| 8.31 | 391 | 0.25 | 10.63 | 22.4 |
| 9.71 | 403 | 0.18 | 9.10 | 23.1 |
| 10.50 | 789 | 0.39 | 8.42 | 45.3 |
| 11.96 | 394 | 0.12 | 7.39 | 22.6 |
| 12.69 | 1033 | 0.38 | 6.97 | 59.3 |
| 13.03 | 533 | 0.44 | 6.79 | 30.6 |
| 13.40 | 375 | 0.15 | 6.60 | 21.5 |
| 15.45 | 1743 | 0.34 | 5.73 | 100 |
| 16.02 | 987 | 0.37 | 5.53 | 56.6 |
| 16.60 | 825 | 0.30 | 5.34 | 47.3 |
| 17.50 | 532 | 0.16 | 5.06 | 30.5 |
| 19.12 | 1052 | 0.34 | 4.64 | 60.4 |
| 20.66 | 1196 | 0.65 | 4.30 | 68.6 |
| 21.39 | 1317 | 0.71 | 4.15 | 75.6 |
| 22.28 | 1422 | 0.50 | 3.99 | 81.6 |
| 22.75 | 931 | 0.45 | 3.90 | 53.4 |
| 24.13 | 589 | 0.27 | 3.68 | 33.8 |
| 24.93 | 740 | 0.19 | 3.57 | 42.5 |
| 25.26 | 633 | 1.16 | 3.52 | 36.3 |
| 25.95 | 613 | 0.54 | 3.43 | 35.2 |
| 27.13 | 540 | 0.33 | 3.28 | 31 |

The present disclosure further provides a crystal form B of the compound of formula (I), wherein the crystal form B has an X-ray powder diffraction pattern comprising characteristic peaks at the following 2Θ angles: 6.96 ± 0.2°, 11.71 ± 0.2°, 15.55 ± 0.2°, 17.35 ± 0.2°, 20.62 ± 0.2°, 21.16 ± 0.2°.

In some embodiments of the present disclosure, the X-ray powder diffraction pattern of the crystal form B comprises characteristic peaks at the following 2Θ angles: 6.96 ± 0.2°, 10.80 ± 0.2°, 11.71 ± 0.2°, 13.39 ± 0.2°, 15.55 ± 0.2°, 17.35 ± 0.2°, 18.45 ± 0.2°, 20.62 ± 0.2°, 21.16 ± 0.2°, 21.81 ± 0.2°.

In some embodiments of the present disclosure, the X-ray powder diffraction pattern of the crystal form B is an X-ray powder diffraction pattern basically as shown in Fig. 2.

In some embodiments of the present disclosure, analytical data for the X-ray powder diffraction pattern of the crystal form B is as shown in Table 2.

**Table 2**

| Pos. [°2Th.] | Height [cts] | FWHM Left [°2Th.] | d-spacing [Å] | Rel. Int. [%] |
|---|---|---|---|---|
| 5.90 | 56.28 | 0.31 | 14.97 | 10.12 |
| 6.96 | 133.17 | 0.31 | 12.69 | 23.94 |
| 8.43 | 62.90 | 0.31 | 10.49 | 11.31 |
| 10.80 | 110.78 | 0.24 | 8.19 | 19.92 |
| 11.71 | 202.82 | 0.39 | 7.56 | 36.47 |
| 13.39 | 125.43 | 0.39 | 6.61 | 22.55 |
| 15.55 | 256.69 | 0.20 | 5.70 | 46.15 |
| 17.35 | 323.49 | 0.87 | 5.11 | 58.16 |
| 18.45 | 131.46 | 0.24 | 4.81 | 23.64 |
| 20.62 | 556.17 | 0.28 | 4.31 | 100.00 |
| 21.16 | 530.27 | 0.35 | 4.20 | 95.34 |
| 21.81 | 266.83 | 0.39 | 4.08 | 47.98 |
| 23.15 | 130.52 | 0.47 | 3.84 | 23.47 |
| 24.49 | 250.34 | 0.24 | 3.64 | 45.01 |
| 25.49 | 250.04 | 0.39 | 3.49 | 44.96 |
| 33.11 | 11.62 | 0.94 | 2.71 | 2.09 |

The present disclosure further provides a crystal form C of the compound of formula (I), wherein the crystal form C has an X-ray powder diffraction pattern comprising characteristic peaks at the following 2θ angles: 12.69 ± 0.2°, 14.38 ± 0.2°, 16.23 ± 0.2°, 17.40 ± 0.2°, 18.61 ± 0.2°, 19.74 ± 0.2°.

In some embodiments of the present disclosure, the X-ray powder diffraction pattern of the crystal form C comprises characteristic peaks at the following 2θ angles: 12.69 ± 0.2°, 14.38 ± 0.2°, 16.23 ± 0.2°, 17.40 ± 0.2°, 18.61 ± 0.2°, 19.74 ± 0.2°, 20.46 ± 0.2°, 20.94 ± 0.2°, 21.75 ± 0.2°, 25.44 ± 0.2°.

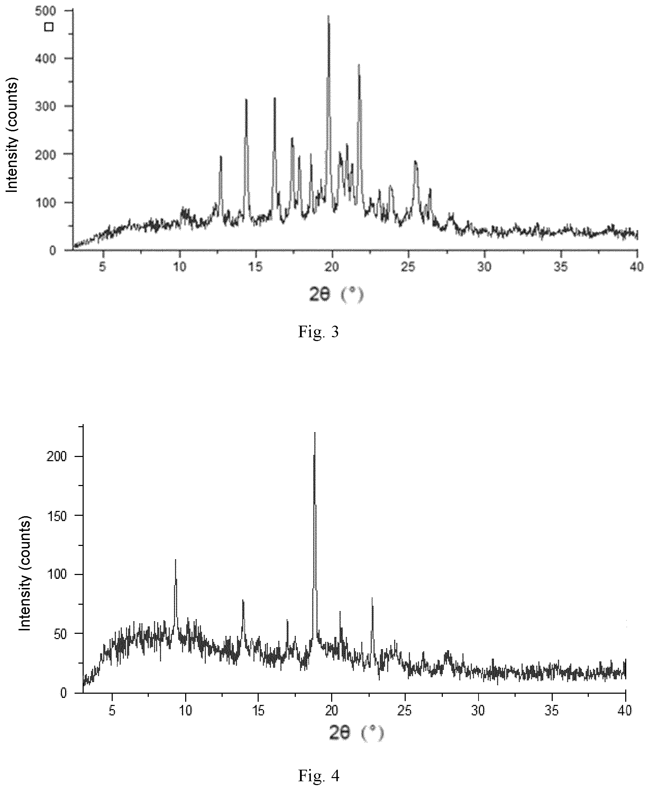

In some embodiments of the present disclosure, the X-ray powder diffraction pattern of the crystal form C is an X-ray powder diffraction pattern basically as shown in Fig. 3.

In some embodiments of the present disclosure, analytical data for the X-ray powder diffraction pattern of the crystal form C is as shown in Table 3.

**Table 3**

| Pos. [°2Th.] | Height [cts] | FWHM Left [°2Th.] | d-spacing [Å] | Rel. Int. [%] |
|---|---|---|---|---|
| 10.34 | 20.18 | 0.47 | 8.56 | 4.56 |
| 12.69 | 142.83 | 0.14 | 6.97 | 32.30 |
| 14.38 | 266.87 | 0.12 | 6.16 | 60.35 |
| 16.23 | 249.54 | 0.10 | 5.46 | 56.44 |
| 17.40 | 166.61 | 0.14 | 5.10 | 37.68 |
| 17.86 | 138.54 | 0.12 | 4.97 | 31.33 |
| 18.61 | 158.32 | 0.12 | 4.77 | 35.80 |
| 19.74 | 442.17 | 0.14 | 4.50 | 100.00 |
| 20.46 | 146.85 | 0.20 | 4.34 | 33.21 |
| 20.94 | 158.72 | 0.12 | 4.24 | 35.90 |
| 21.29 | 131.16 | 0.12 | 4.17 | 29.66 |
| 21.75 | 336.65 | 0.12 | 4.09 | 76.14 |
| 23.09 | 80.23 | 0.16 | 3.85 | 18.15 |
| 23.80 | 91.72 | 0.12 | 3.74 | 20.74 |
| 25.44 | 144.65 | 0.28 | 3.50 | 32.71 |
| 26.41 | 81.60 | 0.12 | 3.37 | 18.45 |
| 27.74 | 26.82 | 0.39 | 3.22 | 6.07 |

The present disclosure further provides a crystal form D of the compound of formula (I), wherein the crystal form D has an X-ray powder diffraction pattern comprising characteristic peaks at the following 2θ angles: 9.33 ± 0.2°, 13.94 ± 0.2°, 18.82 ± 0.2°, 22.76 ± 0.2°, 24.24 ± 0.2°, 27.90 ± 0.2°.

In some embodiments of the present disclosure, the X-ray powder diffraction pattern of the crystal form D is an X-ray powder diffraction pattern basically as shown in Fig. 4.

In some embodiments of the present disclosure, analytical data for the X-ray powder diffraction pattern of the crystal form D is as shown in Table 4.

**Table 4**

| Pos. [°2Th.] | Height [cts] | FWHM Left [°2Th.] | d-spacing [Å] | Rel. Int. [%] |
|---|---|---|---|---|
| 4.65 | 8.44 | 0.94 | 19.01 | 4.53 |
| 9.33 | 61.21 | 0.12 | 9.48 | 32.85 |
| 13.94 | 45.87 | 0.16 | 6.35 | 24.61 |
| 17.19 | 6.60 | 0.79 | 5.16 | 3.54 |
| 18.82 | 186.36 | 0.12 | 4.71 | 100.00 |
| 22.76 | 49.61 | 0.16 | 3.91 | 26.62 |
| 24.24 | 13.02 | 0.94 | 3.67 | 6.98 |
| 27.90 | 14.03 | 0.47 | 3.20 | 7.53 |

The present disclosure further provides a crystal form E of the compound of formula (I), wherein the crystal form E has an X-ray powder diffraction pattern comprising characteristic peaks at the following 2θ angles: 6.84 ± 0.2°, 10.65 ± 0.2°, 11.53 ± 0.2°, 15.41 ± 0.2°, 17.29 ± 0.2°, 20.65 ± 0.2°.

In some embodiments of the present disclosure, the X-ray powder diffraction pattern of the crystal form E comprises characteristic peaks at the following 2Θ angles: 6.84 ± 0.2°, 10.65 ± 0.2°, 11.53 ± 0.2°, 13.78 ± 0.2°, 15.41 ± 0.2°, 17.29 ± 0.2°, 20.22 ± 0.2°, 20.65 ± 0.2°, 23.90 ± 0.2°, 25.26 ± 0.2°.

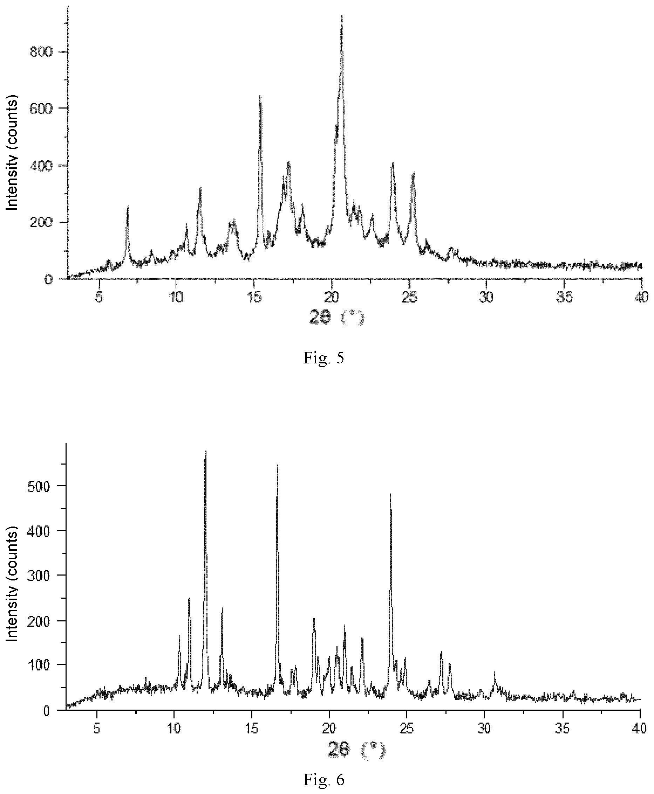

In some embodiments of the present disclosure, the X-ray powder diffraction pattern of the crystal form E is an X-ray powder diffraction pattern basically as shown in Fig. 5.

In some embodiments of the present disclosure, analytical data for the X-ray powder diffraction pattern of the crystal form E is as shown in Table 5.

**Table 5**

| Pos. [°2Th.] | Height [cts] | FWHM Left [°2Th.] | d-spacing [Å] | Rel. Int. [%] |
|---|---|---|---|---|
| 6.84 | 213.57 | 0.12 | 12.93 | 24.96 |
| 8.34 | 46.17 | 0.24 | 10.60 | 5.40 |
| 10.65 | 131.82 | 0.12 | 8.31 | 15.41 |
| 11.53 | 275.98 | 0.08 | 7.67 | 32.26 |
| 13.78 | 130.09 | 0.55 | 6.42 | 15.21 |
| 15.41 | 568.65 | 0.18 | 5.75 | 66.47 |
| 17.29 | 317.03 | 0.16 | 5.13 | 37.06 |
| 18.12 | 193.21 | 0.12 | 4.90 | 22.58 |
| 20.22 | 441.18 | 0.12 | 4.39 | 51.57 |
| 20.65 | 855.52 | 0.08 | 4.30 | 100.00 |
| 21.85 | 173.38 | 0.24 | 4.07 | 20.27 |
| 22.64 | 159.84 | 0.24 | 3.93 | 18.68 |
| 23.90 | 347.11 | 0.14 | 3.72 | 40.57 |
| 25.26 | 309.61 | 0.24 | 3.53 | 36.19 |
| 27.65 | 44.83 | 0.24 | 3.23 | 5.24 |

The present disclosure further provides a diethylamine salt of the compound of formula (I).

The present disclosure further provides a crystal form F of the diethylamine salt of the compound of formula (I), wherein the crystal form F has an X-ray powder diffraction pattern comprising characteristic peaks at the following 2θ angles: 10.36 ± 0.2°, 10.96 ± 0.2°, 12.01 ± 0.2°, 13.05 ± 0.2°, 16.66 ± 0.2°, 18.99 ± 0.2°.

In some embodiments of the present disclosure, the X-ray powder diffraction pattern of the crystal form F comprises characteristic peaks at the following 2θ angles: 10.36 ± 0.2°, 10.96 ± 0.2°, 12.01 ± 0.2°, 13.05 ± 0.2°, 16.66 ± 0.2°, 18.99 ± 0.2°, 20.42 ± 0.2°, 20.98 ± 0.2°, 22.11 ± 0.2°, 23.93 ± 0.2°.

In some embodiments of the present disclosure, the X-ray powder diffraction pattern of the crystal form F is an X-ray powder diffraction pattern basically as shown in Fig. 6.

In some embodiments of the present disclosure, analytical data for the X-ray powder diffraction pattern of the crystal form F is as shown in Table 6.

**Table 6**

| Pos. [°2Th.] | Height [cts] | FWHM Left [°2Th.] | d-spacing [Å] | Rel. Int. [%] |
|---|---|---|---|---|
| 10.36 | 119.43 | 0.10 | 8.54 | 22.45 |
| 10.96 | 200.05 | 0.10 | 8.07 | 37.60 |
| 12.01 | 532.05 | 0.10 | 7.37 | 100.00 |
| 13.05 | 168.95 | 0.12 | 6.79 | 31.75 |
| 16.66 | 514.45 | 0.10 | 5.32 | 96.69 |
| 17.86 | 57.46 | 0.14 | 4.97 | 10.80 |
| 18.99 | 161.92 | 0.12 | 4.67 | 30.43 |
| 19.27 | 74.44 | 0.12 | 4.61 | 13.99 |
| 19.96 | 80.73 | 0.12 | 4.45 | 15.17 |
| 20.42 | 85.76 | 0.14 | 4.35 | 16.12 |
| 20.98 | 140.17 | 0.12 | 4.23 | 26.35 |
| 21.43 | 61.04 | 0.12 | 4.15 | 11.47 |
| 22.11 | 122.11 | 0.14 | 4.02 | 22.95 |
| 23.93 | 446.60 | 0.14 | 3.72 | 83.94 |
| 24.88 | 82.80 | 0.16 | 3.58 | 15.56 |
| 26.38 | 27.06 | 0.24 | 3.38 | 5.09 |
| 27.20 | 96.56 | 0.16 | 3.28 | 18.15 |
| 27.73 | 74.63 | 0.14 | 3.22 | 14.03 |
| 30.64 | 45.36 | 0.16 | 2.92 | 8.53 |
| 35.71 | 12.05 | 0.47 | 2.51 | 2.26 |
| 39.02 | 6.96 | 0.31 | 2.31 | 1.31 |

The present disclosure further provides a crystal form H of the calcium salt of the compound of formula (I), wherein the crystal form H has an X-ray powder diffraction pattern comprising characteristic peaks at the following 2θ angles: 7.32 ± 0.2°, 9.57 ± 0.2°, 11.83 ± 0.2°, 12.76 ± 0.2°, 13.48 ± 0.2°, 14.83 ± 0.2°.

In some embodiments of the present disclosure, the X-ray powder diffraction pattern of the crystal form H comprises characteristic peaks at the following 2θ angles: 7.32 ± 0.2°, 9.57 ± 0.2°, 11.83 ± 0.2°, 12.76 ± 0.2°, 13.48 ± 0.2°, 14.83 ± 0.2°, 16.64 ± 0.2°, 17.69 ± 0.2°, 18.91 ± 0.2°, 21.33 ± 0.2°.

In some embodiments of the present disclosure, the X-ray powder diffraction pattern of the crystal form H is an X-ray powder diffraction pattern basically as shown in Fig. 8.

In some embodiments of the present disclosure, analytical data for the X-ray powder diffraction pattern of the crystal form H is as shown in Table 8.

**Table 8**

| Pos. [°2Th.] | Height [cts] | FWHM Left [°2Th.] | d-spacing [Å] | Rel. Int. [%] |
|---|---|---|---|---|
| 7.32 | 31.92 | 0.47 | 12.07 | 27.57 |
| 9.57 | 72.38 | 0.12 | 9.24 | 62.52 |
| 11.83 | 42.53 | 0.24 | 7.48 | 36.73 |
| 12.76 | 115.78 | 0.28 | 6.94 | 100.00 |
| 13.48 | 104.78 | 0.10 | 6.57 | 90.51 |
| 14.83 | 56.68 | 0.31 | 5.97 | 48.96 |
| 16.64 | 42.14 | 0.31 | 5.33 | 36.40 |
| 17.69 | 40.85 | 0.47 | 5.01 | 35.28 |
| 18.91 | 71.48 | 0.16 | 4.69 | 61.74 |
| 21.33 | 92.62 | 0.16 | 4.17 | 80.00 |
| 23.78 | 67.60 | 0.12 | 3.74 | 58.39 |

The present disclosure further provides a crystal form I of the calcium salt of the compound of formula (I), wherein the crystal form I has an X-ray powder diffraction pattern comprising characteristic peaks at the following 2θ angles: 7.27 ± 0.2°, 8.93 ± 0.2°, 11.23 ± 0.2°, 12.34 ± 0.2°, 13.66 ± 0.2°, 16.06 ± 0.2°.

In some embodiments of the present disclosure, the X-ray powder diffraction pattern of the crystal form I comprises characteristic peaks at the following 2θ angles: 7.27 ± 0.2°, 8.93 ± 0.2°, 11.23 ± 0.2°, 12.34 ± 0.2°, 13.66 ± 0.2°, 16.06 ± 0.2°, 17.97 ± 0.2°, 20.82 ± 0.2°, 23.42 ± 0.2°, 24.38 ± 0.2°.

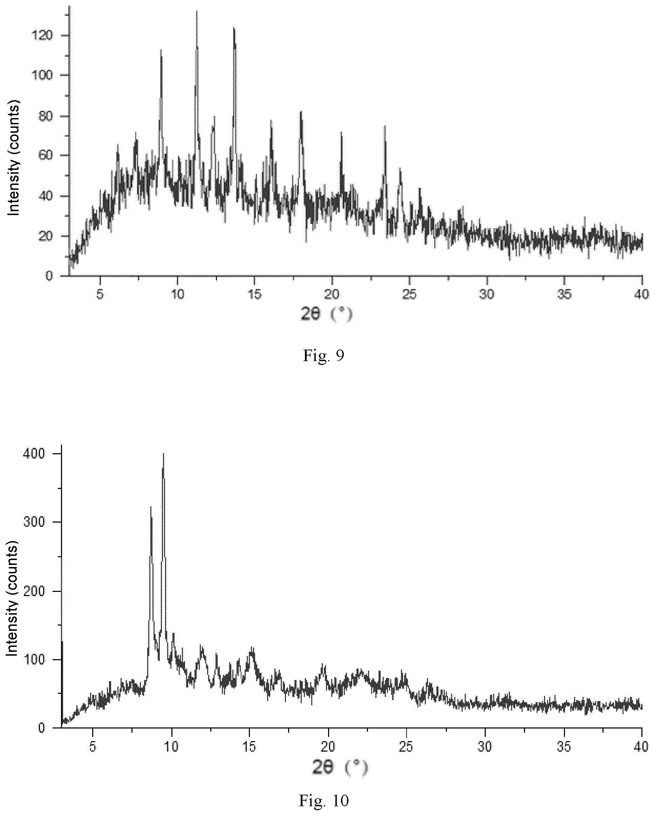

In some embodiments of the present disclosure, the X-ray powder diffraction pattern of the crystal form I is an X-ray powder diffraction pattern basically as shown in Fig. 9.

In some embodiments of the present disclosure, analytical data for the X-ray powder diffraction pattern of the crystal form I is as shown in Table 9.

**Table 9**

| Pos. [°2Th.] | Height [cts] | FWHM Left [°2Th.] | d-spacing [Å] | Rel. Int. [%] |
|---|---|---|---|---|
| 7.27 | 21.18 | 0.24 | 12.16 | 23.93 |
| 8.93 | 67.89 | 0.12 | 9.90 | 76.70 |
| 11.23 | 80.80 | 0.16 | 7.88 | 91.29 |
| 12.34 | 37.44 | 0.20 | 7.17 | 42.30 |
| 13.66 | 88.51 | 0.12 | 6.48 | 100.00 |
| 16.06 | 37.63 | 0.16 | 5.52 | 42.52 |
| 17.97 | 42.64 | 0.20 | 4.94 | 48.17 |
| 20.82 | 9.74 | 0.94 | 4.27 | 11.00 |
| 23.42 | 36.68 | 0.12 | 3.80 | 41.44 |
| 24.38 | 23.10 | 0.24 | 3.65 | 26.10 |

The present disclosure further provides a crystal form J of the calcium salt of the compound of formula (I), wherein the crystal form J has an X-ray powder diffraction pattern comprising characteristic peaks at the following 2θ angles: 8.73 ± 0.2°, 9.49 ± 0.2°, 11.99 ± 0.2°, 12.89 ± 0.2°, 15.16 ± 0.2°, 19.58 ± 0.2°.

In some embodiments of the present disclosure, the X-ray powder diffraction pattern of the crystal form J comprises characteristic peaks at the following 2 angles: 4.52 ± 0.2°, 8.73 ± 0.2°, 9.49 ± 0.2°, 11.99 ± 0.2°, 12.89 ± 0.2°, 15.16 ± 0.2°, 19.58 ± 0.2°, 21.96 ± 0.2°, 24.84 ± 0.2°, 26.66 ± 0.2°.

In some embodiments of the present disclosure, the X-ray powder diffraction pattern of the crystal form J is an X-ray powder diffraction pattern basically as shown in Fig. 10.

In some embodiments of the present disclosure, analytical data for the X-ray powder diffraction pattern of the crystal form J is as shown in Table 10.

**Table 10**

| Pos. [°2Th.] | Height [cts] | FWHM Left [°2Th.] | d-spacing [Å] | Rel. Int. [%] |
|---|---|---|---|---|
| 4.52 | 6.35 | 0.94 | 19.54 | 1.85 |
| 8.73 | 230.64 | 0.14 | 10.13 | 67.36 |
| 9.49 | 342.39 | 0.18 | 9.32 | 100.00 |
| 11.99 | 52.90 | 0.63 | 7.38 | 15.45 |
| 12.89 | 39.80 | 0.24 | 6.87 | 11.63 |
| 15.16 | 51.84 | 0.47 | 5.85 | 15.14 |
| 19.58 | 33.83 | 0.47 | 4.53 | 9.88 |
| 21.96 | 30.00 | 0.94 | 4.05 | 8.76 |
| 24.84 | 26.40 | 0.63 | 3.58 | 7.71 |
| 26.66 | 11.47 | 0.94 | 3.34 | 3.35 |

The present disclosure further provides a crystal form K of the calcium salt of the compound of formula (I), wherein the crystal form K has an X-ray powder diffraction pattern comprising characteristic peaks at the following 2θ angles: 8.57 ± 0.2°, 9.32 ± 0.2°, 11.06 ± 0.2°, 12.65 ± 0.2°, 14.08 ± 0.2°, 14.71 ± 0.2°.

In some embodiments of the present disclosure, the X-ray powder diffraction pattern of the crystal form K comprises characteristic peaks at the following 2θ angles: 7.66 ± 0.2°, 8.57 ± 0.2°, 9.32 ± 0.2°, 11.06 ± 0.2°, 12.65 ± 0.2°, 14.08 ± 0.2°, 14.71 ± 0.2°, 15.08 ± 0.2°, 15.79 ± 0.2°, 19.29 ± 0.2°.

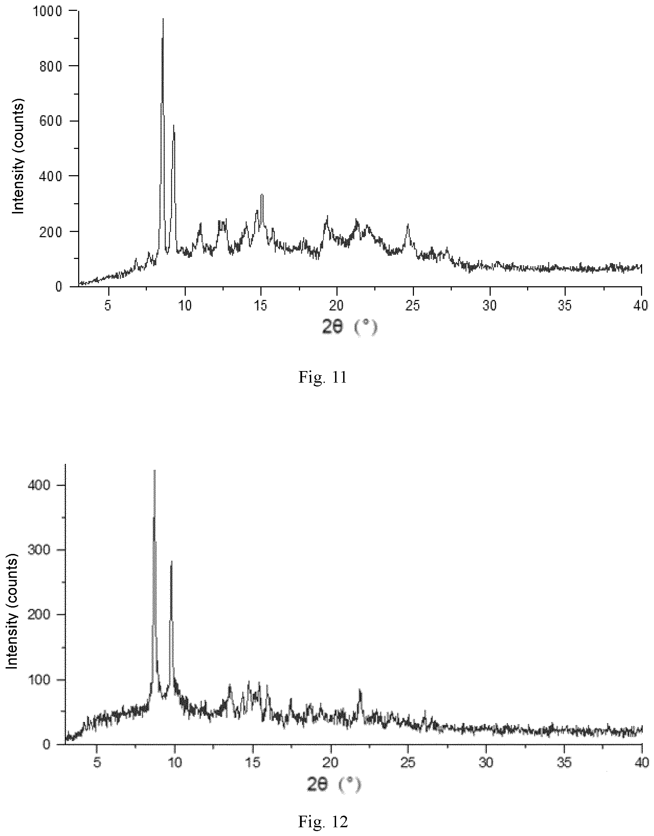

In some embodiments of the present disclosure, the X-ray powder diffraction pattern of the crystal form K is an X-ray powder diffraction pattern basically as shown in Fig. 11.

In some embodiments of the present disclosure, analytical data for the X-ray powder diffraction pattern of the crystal form K is as shown in Table 11.

**Table 11**

| Pos. [°2Th.] | Height [cts] | FWHM Left [°2Th.] | d-spacing [Å] | Rel. Int. [%] |
|---|---|---|---|---|
| 6.82 | 53.61 | 0.16 | 12.96 | 5.97 |
| 7.66 | 67.14 | 0.24 | 11.54 | 7.48 |
| 8.57 | 897.97 | 0.16 | 10.31 | 100.00 |
| 9.32 | 472.14 | 0.16 | 9.49 | 52.58 |
| 11.06 | 129.01 | 0.35 | 8.00 | 14.37 |
| 12.65 | 138.66 | 0.55 | 7.00 | 15.44 |
| 14.08 | 119.10 | 0.24 | 6.29 | 13.26 |
| 14.71 | 176.56 | 0.16 | 6.02 | 19.66 |
| 15.08 | 232.49 | 0.12 | 5.87 | 25.89 |
| 15.79 | 112.77 | 0.12 | 5.61 | 12.56 |
| 17.85 | 38.97 | 0.47 | 4.97 | 4.34 |
| 19.29 | 107.59 | 0.31 | 4.60 | 11.98 |
| 21.31 | 83.59 | 0.24 | 4.17 | 9.31 |
| 24.67 | 107.77 | 0.16 | 3.61 | 12.00 |

The present disclosure further provides a crystal form L of the calcium salt of the compound of formula (I), wherein the crystal form L has an X-ray powder diffraction pattern comprising characteristic peaks at the following 2θ angles: 8.69 ± 0.2°, 9.78 ± 0.2°, 13.46 ± 0.2°, 14.72 ± 0.2°, 15.40 ± 0.2°, 15.98 ± 0.2°.

In some embodiments of the present disclosure, the X-ray powder diffraction pattern of the crystal form L is an X-ray powder diffraction pattern basically as shown in Fig. 12.

In some embodiments of the present disclosure, analytical data for the X-ray powder diffraction pattern of the crystal form L is as shown in Table 12.

**Table 12**

| Pos. [°2Th.] | Height [cts] | FWHM Left [°2Th.] | d-spacing [Å] | Rel. Int. [%] |
|---|---|---|---|---|
| 8.69 | 352.70 | 0.12 | 10.17 | 100.00 |
| 9.78 | 232.10 | 0.12 | 9.04 | 65.80 |
| 13.46 | 39.74 | 0.28 | 6.58 | 11.27 |
| 14.72 | 50.20 | 0.16 | 6.02 | 14.23 |
| 15.40 | 42.16 | 0.24 | 5.75 | 11.95 |
| 15.98 | 36.71 | 0.24 | 5.55 | 10.41 |
| 17.47 | 21.47 | 0.24 | 5.08 | 6.09 |
| 18.68 | 20.43 | 0.24 | 4.75 | 5.79 |
| 21.91 | 39.27 | 0.24 | 4.06 | 11.13 |

The present disclosure further provides a crystal form M of the calcium salt of the compound of formula (I), wherein the crystal form M has an X-ray powder diffraction pattern comprising characteristic peaks at the following 2θ angles: 8.09 ± 0.2°, 10.87 ± 0.2°, 13.62 ± 0.2°, 14.55 ± 0.2°, 15.77 ± 0.2°, 16.39 ± 0.2°.

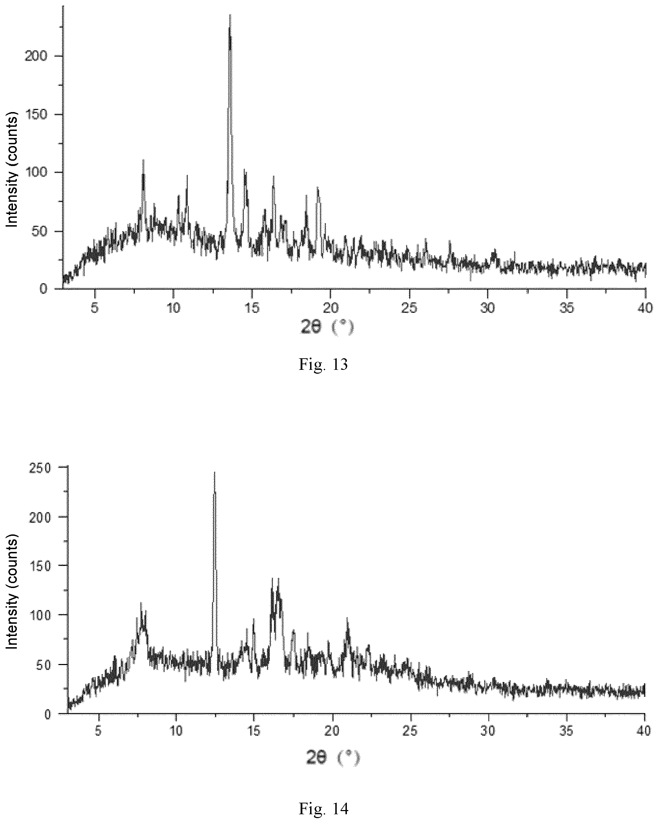

In some embodiments of the present disclosure, the X-ray powder diffraction pattern of the crystal form M is an X-ray powder diffraction pattern basically as shown in Fig. 13.

In some embodiments of the present disclosure, analytical data for the X-ray powder diffraction pattern of the crystal form M is as shown in Table 13.

**Table 13**

| Pos. [°2Th.] | Height [cts] | FWHM Left [°2Th.] | d-spacing [Å] | Rel. Int. [%] |
|---|---|---|---|---|
| 8.09 | 62.44 | 0.12 | 10.93 | 35.92 |
| 10.87 | 41.97 | 0.24 | 8.14 | 24.14 |
| 13.62 | 173.83 | 0.14 | 6.50 | 100.00 |
| 14.55 | 63.24 | 0.16 | 6.09 | 36.38 |
| 15.77 | 31.49 | 0.16 | 5.62 | 18.12 |
| 16.39 | 55.45 | 0.16 | 5.41 | 31.90 |
| 18.44 | 39.68 | 0.16 | 4.81 | 22.83 |
| 19.19 | 52.13 | 0.24 | 4.63 | 29.99 |
| 30.42 | 11.82 | 0.63 | 2.94 | 6.80 |

The present disclosure further provides a crystal form N of the calcium salt of the compound of formula (I), wherein the crystal form N has an X-ray powder diffraction pattern comprising characteristic peaks at the following 2θ angles: 7.79 ± 0.2°, 12.44 ± 0.2°, 14.94 ± 0.2°, 16.11 ± 0.2°, 17.50 ± 0.2°, 20.98 ± 0.2°.

In some embodiments of the present disclosure, the X-ray powder diffraction pattern of the crystal form N is an X-ray powder diffraction pattern basically as shown in Fig. 14.

In some embodiments of the present disclosure, analytical data for the X-ray powder diffraction pattern of the crystal form N is as shown in Table 14.

**Table 14**

| Pos. [°2Th.] | Height [cts] | FWHM Left [°2Th.] | d-spacing [Å] | Rel. Int. [%] |
|---|---|---|---|---|
| 7.79 | 45.29 | 0.47 | 11.34 | 24.17 |
| 12.44 | 187.41 | 0.14 | 7.12 | 100.00 |
| 14.48 | 14.97 | 0.94 | 6.12 | 7.99 |
| 14.94 | 40.33 | 0.16 | 5.93 | 21.52 |
| 16.11 | 73.38 | 0.16 | 5.50 | 39.15 |
| 16.58 | 62.66 | 0.31 | 5.35 | 33.43 |
| 17.50 | 34.62 | 0.20 | 5.07 | 18.47 |
| 20.98 | 30.52 | 0.24 | 4.23 | 16.29 |

The present disclosure further provides a crystal form O of the calcium salt of the compound of formula (I), wherein the crystal form O has an X-ray powder diffraction pattern comprising characteristic peaks at the following 2θ angles: 7.40 ± 0.2°, 8.52 ± 0.2°, 12.01 ± 0.2°, 12.24 ± 0.2°, 14.67 ± 0.2°, 16.86 ± 0.2°.

In some embodiments of the present disclosure, the X-ray powder diffraction pattern of the crystal form O comprises characteristic peaks at the following 2θ angles: 7.40 ± 0.2°, 8.52 ± 0.2°, 12.01 ± 0.2°, 12.24 ± 0.2°, 14.67 ± 0.2°, 15.35 ± 0.2°, 16.86 ± 0.2°, 18.05 ± 0.2°, 19.77 ± 0.2°, 22.02 ± 0.2°.

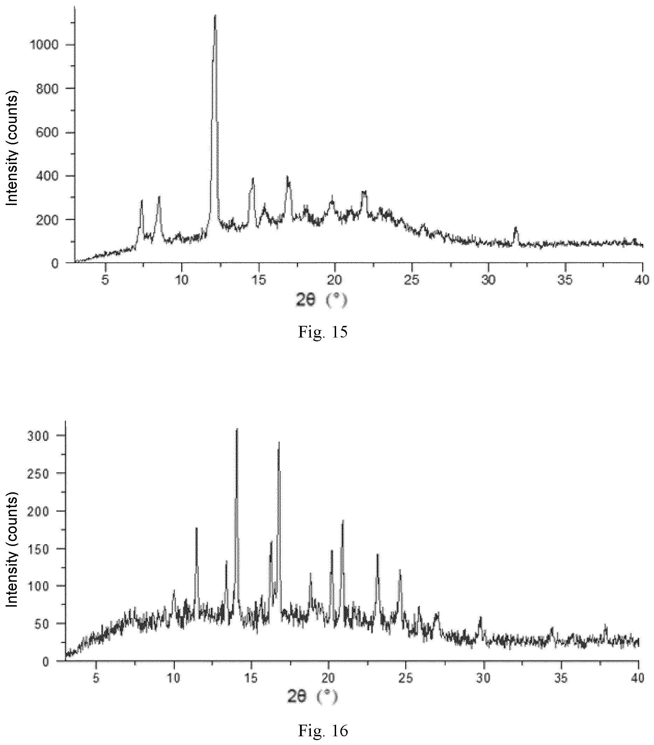

In some embodiments of the present disclosure, the X-ray powder diffraction pattern of the crystal form O is an X-ray powder diffraction pattern basically as shown in Fig. 15.

In some embodiments of the present disclosure, analytical data for the X-ray powder diffraction pattern of the crystal form O is as shown in Table 15.

**Table 15**

| Pos. [°2Th.] | Height [cts] | FWHM Left [°2Th.] | d-spacing [Å] | Rel. Int. [%] |
|---|---|---|---|---|
| 7.40 | 215.39 | 0.16 | 11.94 | 25.21 |
| 8.52 | 219.73 | 0.16 | 10.38 | 25.72 |
| 12.01 | 740.02 | 0.08 | 7.37 | 86.62 |
| 12.24 | 854.29 | 0.16 | 7.23 | 100.00 |
| 14.67 | 240.13 | 0.14 | 6.04 | 28.11 |
| 15.35 | 132.30 | 0.39 | 5.77 | 15.49 |
| 16.86 | 270.37 | 0.20 | 5.26 | 31.65 |
| 18.05 | 121.69 | 0.24 | 4.91 | 14.25 |
| 19.77 | 169.85 | 0.31 | 4.49 | 19.88 |
| 22.02 | 184.58 | 0.18 | 4.04 | 21.61 |
| 23.52 | 105.48 | 0.79 | 3.78 | 12.35 |
| 25.72 | 48.75 | 0.39 | 3.46 | 5.71 |
| 31.80 | 68.93 | 0.24 | 2.81 | 8.07 |

The present disclosure further provides a crystal form P of the calcium salt of the compound of formula (I), wherein the crystal form P has an X-ray powder diffraction pattern comprising characteristic peaks at the following 2θ angles: 11.49 ± 0.2°, 13.38 ± 0.2°, 14.04 ± 0.2°, 16.28 ± 0.2°, 16.78 ± 0.2°, 20.22 ± 0.2°, 20.86 ± 0.2°.

In some embodiments of the present disclosure, the X-ray powder diffraction pattern of the crystal form P comprises characteristic peaks at the following 2θ angles: 11.49 ± 0.2°, 13.38 ± 0.2°, 14.04 ± 0.2°, 16.28 ± 0.2°, 16.78 ± 0.2°, 18.81 ± 0.2°, 20.22 ± 0.2°, 20.86 ± 0.2°, 23.16 ± 0.2°, 24.66 ± 0.2°, 25.87 ± 0.2°.

In some embodiments of the present disclosure, the X-ray powder diffraction pattern of the crystal form P is an X-ray powder diffraction pattern basically as shown in Fig. 16.

In some embodiments of the present disclosure, analytical data for the X-ray powder diffraction pattern of the crystal form P is as shown in Table 16.

**Table 16**

| Pos. [°2Th.] | Height [cts] | FWHM Left [°2Th.] | d-spacing [Å] | Rel. Int. [%] |
|---|---|---|---|---|
| 9.98 | 36.08 | 0.24 | 8.86 | 13.70 |
| 11.49 | 124.53 | 0.10 | 7.70 | 47.30 |
| 13.38 | 81.27 | 0.12 | 6.62 | 30.87 |
| 14.04 | 263.29 | 0.10 | 6.31 | 100.00 |
| 16.28 | 99.48 | 0.14 | 5.45 | 37.78 |
| 16.78 | 248.41 | 0.12 | 5.28 | 94.35 |
| 18.81 | 70.34 | 0.12 | 4.72 | 26.72 |
| 20.22 | 113.07 | 0.10 | 4.39 | 42.95 |
| 20.86 | 139.00 | 0.12 | 4.26 | 52.79 |
| 23.16 | 109.05 | 0.16 | 3.84 | 41.42 |
| 24.66 | 77.38 | 0.14 | 3.61 | 29.39 |
| 25.87 | 31.41 | 0.24 | 3.44 | 11.93 |
| 27.06 | 29.62 | 0.35 | 3.29 | 11.25 |
| 29.72 | 21.75 | 0.24 | 3.01 | 8.26 |
| 34.38 | 13.56 | 0.47 | 2.61 | 5.15 |

The present disclosure further provides a strontium salt of the compound of formula (I).

In some embodiments of the present disclosure, a crystal form Q of the strontium salt of the compound of formula (I) is provided, wherein the crystal form Q has an X-ray powder diffraction pattern comprising characteristic peaks at the following 2θ angles: 5.01 ± 0.2°, 8.96 ± 0.2°, 10.08 ± 0.2°, 13.54 ± 0.2°, 14.84 ± 0.2°, 24.67 ± 0.2°.

In some embodiments of the present disclosure, the X-ray powder diffraction pattern of the crystal form Q comprises characteristic peaks at the following 2θ angles: 5.01 ± 0.2°, 8.96 ± 0.2°, 10.08 ± 0.2°, 13.54 ± 0.2°, 14.84 ± 0.2°, 18.43 ± 0.2°, 21.10 ± 0.2°, 22.10 ± 0.2°, 23.80 ± 0.2°, 24.67 ± 0.2°.

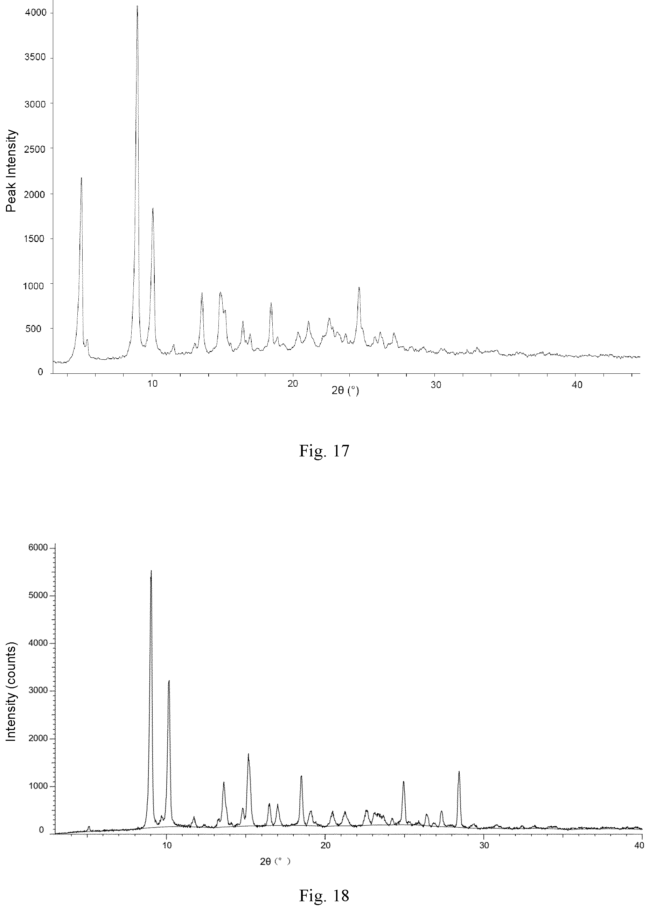

In some embodiments of the present disclosure, the X-ray powder diffraction pattern of the crystal form Q is an X-ray powder diffraction pattern basically as shown in Fig. 17.

In some embodiments of the present disclosure, analytical data for the X-ray powder diffraction pattern of the crystal form Q is as shown in Table 17.

**Table 17**

| Pos. [°2Th.] | Height [cts] | FWHM Left [°2Th.] | d-spacing [Å] | Rel. Int. [%] |
|---|---|---|---|---|
| 5.01 | 2170 | 0.27 | 17.62 | 63.6 |
| 5.41 | 370 | 0.25 | 16.33 | 5.6 |
| 8.96 | 4088 | 0.23 | 9.86 | 100 |
| 10.08 | 1837 | 0.23 | 8.77 | 42.6 |
| 11.55 | 317 | 0.20 | 7.66 | 2.4 |
| 13.05 | 329 | 0.44 | 6.78 | 5.9 |
| 13.54 | 894 | 0.25 | 6.54 | 19.6 |
| 14.84 | 900 | 0.46 | 5.96 | 35.7 |
| 15.20 | 696 | 0.70 | 5.83 | 37 |
| 16.46 | 577 | 0.33 | 5.38 | 12.5 |
| 16.95 | 435 | 0.31 | 5.23 | 6.6 |
| 18.43 | 782 | 0.27 | 4.81 | 15.8 |
| 18.89 | 401 | 0.60 | 4.69 | 9.9 |
| 20.35 | 454 | 0.19 | 4.36 | 3.1 |
| 21.10 | 575 | 0.40 | 4.21 | 12.8 |
| 22.10 | 411 | 0.40 | 4.02 | 4.2 |
| 22.52 | 612 | 0.54 | 3.95 | 18.3 |
| 23.15 | 450 | 0.78 | 3.84 | 7.5 |
| 23.70 | 435 | 0.13 | 3.75 | 1.3 |
| 24.67 | 958 | 0.27 | 3.61 | 19.9 |
| 25.81 | 403 | 0.36 | 3.45 | 4.7 |
| 26.19 | 454 | 0.38 | 3.40 | 7.4 |
| 27.15 | 438 | 0.39 | 3.28 | 7.7 |
| 29.25 | 293 | 0.29 | 3.05 | 1.8 |
| 30.51 | 267 | 0.47 | 2.93 | 2.9 |
| 34.40 | 253 | 0.60 | 2.61 | 3.6 |

The present disclosure further provides a pharmaceutical composition, the pharmaceutical composition comprising the aforementioned crystal form A, crystal form B, crystal form C, crystal form D, crystal form E, diethylamine salt, crystal from F, calcium salt, crystal form G, crystal form H, crystal form I, crystal form J, crystal form K, crystal form L, crystal form M, crystal form N, crystal form O, crystal form P, strontium salt, or crystal form Q.

In some embodiments of the present disclosure, the pharmaceutical composition further comprises a pharmaceutically acceptable carrier, an excipient, a diluent, an adjuvant, a vehicle, or a combination thereof.

The present disclosure further provides a use of the aforementioned crystal form A, crystal form B, crystal form C, crystal form D, crystal form E, diethylamine salt, crystal from F, calcium salt, crystal form G, crystal form H, crystal form I, crystal form J, crystal form K, crystal form L, crystal form M, crystal form N, crystal form O, crystal form P, strontium salt, crystal form Q, or pharmaceutical composition in the manufacture of a medicament for an S1P1 receptor-related disease.

In some embodiments of the present disclosure, the S1P1 receptor-related disease is selected from ulcerative colitis, Crohn's disease, multiple sclerosis, systemic lupus erythematosus, lupus nephritis, rheumatoid arthritis, primary biliary cholangitis, atopic dermatitis, intracerebral hemorrhage, graft-versus-host disease, psoriasis, type I diabetes, acne, microbial infections or microbial diseases, and viral infections or viral diseases.

### Definition and description

Unless otherwise specified, all technical and scientific terms used herein have the same meaning as commonly understood by one of ordinary skill in the art to which the present disclosure belongs. All patents and publications mentioned herein are incorporated by reference in their entireties. Although any methods and materials similar or equivalent to those described herein can be used in the practice or testing of the present disclosure, the preferred methods, devices, and materials are described in the present disclosure.

"Crystal form" or "crystalline form" refers to a solid with a highly regular chemical structure, including but not limited to, a single-component or multi-component crystal, and/or a polymorph, solvate, hydrate, clathrate, co-crystal, salt, solvate of salt, and hydrate of salt of the compound. The crystalline form of a substance can be obtained by numerous methods known in the art. These methods include, but are not limited to, melt crystallization, melt cooling, solvent crystallization, crystallization in a defined space, e.g., in a nanopore or capillary, crystallization on a surface or a template, e.g., on a polymer, crystallization in the presence of an additive such as a co-crystallized antimolecule, desolvation, dehydration, rapid evaporation, rapid cooling, slow cooling, vapor diffusion, sublimation, reactive crystallization, anti-solvent addition, grinding, and solvent-drop grinding.

"Amorphous" or "amorphous form" refers to a substance formed when the particles (molecules, atoms, and ions) of the substance are arranged in three-dimensional space without periodicity, which is characterized by a diffuse X-ray powder diffraction pattern without sharp peaks. Amorphous is a specific physical form of solid matter, with locally ordered structural features suggesting intricate links to crystalline substances. The amorphous form of a substance can be obtained by numerous methods known in the art. These methods include, but are not limited to, quenching, anti-solvent flocculation, ball milling, spray drying, freeze drying, wet granulation, and solid dispersion techniques.

"Solvent" refers to a substance (typically a liquid) that can fully or partially dissolve another substance (typically a solid). The solvents used in the embodiments of the present disclosure include, but are not limited to, water, acetic acid, acetone, acetonitrile, benzene, chloroform, carbon tetrachloride, dichloromethane, dimethyl sulfoxide, 1,4-dioxane, ethanol, ethyl acetate, butanol, *tert*-butanol, *N,N*-dimethylacetamide, *N*,*N*-dimethylformamide, formamide, formic acid, heptane, hexane, isopropanol, methanol, methyl ethyl ketone, 1-methyl-2-pyrrolidone, mesitylene, nitromethane, polyethylene glycol, propanol, 2-propanone, pyridine, tetrahydrofuran, toluene, xylene, and mixtures thereof.

"Antisolvent" refers to a fluid that promotes the precipitation of a product (or product precursor) from a solvent. The antisolvent can include a cold gas, a fluid that promotes precipitation through a chemical reaction, or a fluid that reduces the solubility of the product in the solvent; it may be the same liquid as the solvent but at a different temperature, or it may be a different liquid from the solvent.

"Solvate" refers to a crystal that has a solvent on its surface, within its lattice, or both on its surface and within its lattice, wherein the solvent can be water, acetic acid, acetone, acetonitrile, benzene, chloroform, carbon tetrachloride, dichloromethane, dimethyl sulfoxide, 1,4-dioxane, ethanol, ethyl acetate, butanol, *tert*-butanol, *N*,*N*-dimethylacetamide, *N,N-*dimethylformamide, formamide, formic acid, heptane, hexane, isopropanol, methanol, methyl ethyl ketone, N-methylpyrrolidone, mesitylene, nitromethane, polyethylene glycol, propanol, 2-propanone, pyridine, tetrahydrofuran, toluene, xylene, and mixtures thereof. A specific example of a solvate is a hydrate, in which the solvent on the surface, within the lattice, or both on the surface and within the lattice is water. A hydrate may have, or may not have, solvents other than water on the surface, within the lattice, or both on the surface and within the lattice.

Crystal forms or amorphous forms can be identified using various techniques, such as X-ray powder diffraction (XRPD), infrared absorption spectroscopy (IR), melting point method, differential scanning calorimetry (DSC), thermogravimetric analysis (TGA), nuclear magnetic resonance (NMR), Raman spectroscopy, single-crystal X-ray diffraction, solution calorimetry, scanning electron microscopy (SEM), quantitative analysis, solubility, and dissolution rate, among others.

X-ray powder diffraction (XRPD) can detect changes in crystal forms, crystallinity, crystal structure state, and other information, and is a commonly used method for identifying crystal forms. The peak positions in XRPD patterns primarily depend on the structure of the crystal form and are relatively insensitive to experimental details. In contrast, the relative peak heights depend on many factors related to sample preparation and instrument geometry. Therefore, in some examples, the crystal forms of the present disclosure are characterized by XRPD patterns with specific peak positions, which are basically as shown in the XRPD patterns provided in the accompanying figures of the present disclosure. At the same time, the measurement of 2Θ of XRPD patterns may have experimental error. The measurement of 2Θ of the XRPD patterns may vary slightly between different instruments and samples, and thus the 2Θ values should not be considered absolute. According to the instrument conditions used in the experiments of the present disclosure, the diffraction peaks have an error tolerance of ± 0.2°.

Differential scanning calorimetry (DSC) is a technique that measures the energy difference between a sample and an inert reference material (commonly α-Al₂O₃) as a function of temperature by continuously heating or cooling under the control of a program. The height of the melting peak in a DSC curve depends on many factors related to sample preparation and instrument geometry, while the peak position is relatively insensitive to experimental details. Therefore, in some examples, the crystal forms of the present disclosure are characterized by DSC patterns with characteristic peak positions, which are basically as shown in the DSC patterns provided in the accompanying figures of the present disclosure. At the same time, DSC patterns may have experimental errors. The peak positions and peak values in DSC patterns may vary slightly between different instruments and samples, and thus the peak positions or numerical values of the peak values of the DSC endothermic peaks should not be considered absolute. According to the instrument conditions used in the experiments of the present disclosure, the melting peaks have an error tolerance of ± 3°C.

Solids with the same chemical composition often form polymorph, with different crystal structures under different thermodynamic conditions, also known as variants. This phenomenon is referred to as polymorphism or polytypism. When temperature and pressure conditions change, variants can undergo mutual transformation, a phenomenon known as transformation of crystal form. Due to crystal form transformation, the mechanical, electrical, and magnetic properties of the crystal can change significantly. When the temperature of the crystal form transformation is within the measurable range, this transformation process can be observed on the differential scanning calorimetry (DSC) pattern, which is characterized by the presence of an exothermic peak reflecting this transformation process and simultaneously two or more endothermic peaks, which are characteristic endothermic peaks of different crystal forms before and after the transformation. The crystal forms or amorphous forms of the compounds in the present disclosure can undergo crystal form transformation under appropriate conditions.

Thermogravimetric analysis (TGA) is a technique that determines the mass change of a substance as a function of temperature under the control of a program. It is suitable for examining the loss of solvents in crystals, or the sublimation and decomposition processes of samples, and can infer the presence of crystal water or crystallization solvents in the crystals. The mass change displayed on the TGA curve depends on many factors such as sample preparation and the instrument. The mass change detected by TGA may vary slightly between different instruments and samples. In some examples, the crystal form A of the calcium salt of the present disclosure shows a weight loss of approximately 5.1% at a temperature of about 150°C. According to the instrument conditions used in the experiments of the present disclosure, the mass change has an error tolerance of ± 0.3%.

In the context of the present disclosure, the 2Θ values in the X-ray powder diffraction patterns are expressed in degrees (°).

The term "basically as shown in the figure" means that at least 50%, or at least 60%, or at least 70%, or at least 80%, or at least 90%, or at least 95%, or at least 99% of the peaks in the X-ray powder diffraction pattern, DSC pattern, or TGA results are shown in the figure.

When referring to a pattern or data appearing in the figure, "peak" refers to a feature that can be recognized by those skilled in the art and is not attributable to background noise.

In the context of the present disclosure, when or whether the words "about" or "approximately" are used, it indicates within 10% of a given value or range, suitably within 5%, and particularly within 1%. Alternatively, for those skilled in the art, the terms "about" or "approximately" indicate within an acceptable standard error range of the mean value. Whenever a number with a value of N is disclosed, any number with a value of N+/-1%, N+/-2%, N+/-3%, N+/-5%, N+/-7%, N+/-8%, or N+/-10% or less is expressly disclosed, where "+/" means plus or minus.

The term "comprising" is an open-ended expression, indicating that the specified elements are included but do not exclude other elements.

The salt form, crystal form, or pharmaceutical composition of the present disclosure can be used in the manufacture of a medicament for the S1P1 receptor-related disease. Herein, the S1P1 receptor-related disease is selected from ulcerative colitis, Crohn's disease, multiple sclerosis, systemic lupus erythematosus, lupus nephritis, rheumatoid arthritis, primary biliary cholangitis, atopic dermatitis, intracerebral hemorrhage, graft-versus-host disease, psoriasis, type I diabetes, acne, microbial infections or microbial diseases, and viral infections or viral diseases.

### BRIEF DESCRIPTION OF THE DRAWINGS

Fig. 1 is the X-ray powder diffraction (XRPD) pattern of the crystal form A.
Fig. 2 is the X-ray powder diffraction (XRPD) pattern of the crystal form B.
Fig. 3 is the X-ray powder diffraction (XRPD) pattern of the crystal form C.
Fig. 4 is the X-ray powder diffraction (XRPD) pattern of the crystal form D.
Fig. 5 is the X-ray powder diffraction (XRPD) pattern of the crystal form E.
Fig. 6 is the X-ray powder diffraction (XRPD) pattern of the crystal form F.
Fig. 7 is the X-ray powder diffraction (XRPD) pattern of the crystal form G.
Fig. 8 is the X-ray powder diffraction (XRPD) pattern of the crystal form H.
Fig. 9 is the X-ray powder diffraction (XRPD) pattern of the crystal form I.
Fig. 10 is the X-ray powder diffraction (XRPD) pattern of the crystal form J.
Fig. 11 is the X-ray powder diffraction (XRPD) pattern of the crystal form K.
Fig. 12 is the X-ray powder diffraction (XRPD) pattern of the crystal form L.
Fig. 13 is the X-ray powder diffraction (XRPD) pattern of the crystal form M.
Fig. 14 is the X-ray powder diffraction (XRPD) pattern of the crystal form N.
Fig. 15 is the X-ray powder diffraction (XRPD) pattern of the crystal form O.
Fig. 16 is the X-ray powder diffraction (XRPD) pattern of the crystal form P.
Fig. 17 is the X-ray powder diffraction (XRPD) pattern of the crystal form Q.
Fig. 18 is the X-ray powder diffraction (XRPD) pattern of the crystal form G.
Fig. 19 is the X-ray powder diffraction (XRPD) pattern of the crystal form G.
Fig. 20 is the X-ray powder diffraction (XRPD) pattern of the crystal form G.
Fig. 21 is the X-ray powder diffraction (XRPD) pattern of the crystal form G.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific embodiments have also been disclosed; for one skilled in the art, it is obvious to make various modifications and improvements to the embodiments of the present disclosure without departing from the spirit and scope of the present disclosure.

The X-ray powder diffraction analysis method used in the present disclosure is as follows: X-ray powder diffraction testing was conducted at room temperature using a Bruker D2 PHASER diffractometer (300W low-power X-ray emitter). During the test, a silicon wafer was used to sustain the sample powder, and an X-ray Cu (Ka) tube was used, with a Kα2/Kα1 intensity ratio of 0.50 (1.54439 Å/1.5406 Å). The instrument was set to 30 kV and 10 mA, with a step size of 0.02° (2θ), a scanning time of 0.15 s per step, for a total of 1837 steps.

The differential scanning calorimetry (DSC) analysis method used in the present disclosure is as follows: DSC data were collected using a TA Discovery DSC. The specific method involves weighing several milligrams of the sample into a Tzero aluminum pan, and sealing it with the corresponding Tzero aluminum pan lid. The sealed sample was then heated from room temperature to 300°C at a heating rate of 10°C/min under a nitrogen atmosphere.

The thermogravimetric analysis (TGA) method used in the present disclosure is as follows: TGA data were collected using a TA Discovery TGA. The specific method involves heating several milligrams of the sample from room temperature to 350°C at a heating rate of 10°C/min under a nitrogen atmosphere.

The polarized light microscopy (PLM) analysis method used in the present disclosure is as follows: the sample morphology was analyzed using an Olympus BX53M polarized light microscope at room temperature.

The high-performance liquid chromatography (HPLC) analysis method used in the present disclosure is as follows: an Agilent 1260 was used to test the purity and stability of the sample under the conditions shown in Table 18.

**Table 18: HPLC Purity Method**

| | | | |
|---|---|---|---|
| Instrument | Agilent 1260 HPLC | | |
| Column | Poroshell 120 EC-C18 2.7 µm, 4.6 × 150 mm, P/N: 693975-902 (T) | | |
| Mobile Phase | A: 0.1% phosphoric acid aqueous solution; B: ACN | | |
| Elution Program | Time (min) | A (%) | B (%) |
| | 0.0 | 90 | 10 |
| | 2.0 | 90 | 10 |
| | 8.0 | 20 | 80 |
| | 18.0 | 10 | 90 |
| | 25.0 | 10 | 90 |
| | 25.1 | 90 | 10 |
| | 30.0 | 90 | 10 |
| Run Time | 30 minutes | | |
| Flow Rate | 1.0 mL/min | | |
| Detector | UV 230 nm | | |
| Injection Volume | 5.0 µL | | |
| Sample Room Temperature | Room Temperature | | |
| Column Temperature | 30°C | | |
| Needle Wash Solvent | ACN | | |

The hygroscopicity analysis method used in the present disclosure is as follows: the hygroscopicity of the sample was evaluated using an SMS ADVENTURE DVS at 25°C in a nitrogen atmosphere. Approximately 30 milligrams of the sample were used for the test. The testing methods are as follows:
Method 1 (for anhydrous crystal form):
   increasing from 0% RH to 95% RH in an increment of 10% RH per stage (increasing from 90% RH to 95% RH in an increment of 5% RH);
   decreasing from 95% RH to 0% RH in an increment of 10% RH per stage (decreasing from 95% RH to 90% RH in an increment of 5% RH).
Method 2 (for aqueous co-solvate):
   increasing from ambient humidity to 95% RH in an increment of 10% RH per stage (increasing from 90% RH to 95% RH in an increment of 5% RH);
   decreasing from 95% RH to 0% RH in an increment of 10% RH per stage (decreasing from 95% RH to 90% RH in an increment of 5% RH);
   increasing from 0% RH to 95% RH in an increment of 10% RH per stage (increasing from 90% RH to 95% RH in an increment of 5% RH).

The analytical method for determining the moisture content in the present disclosure is as follows: the moisture content data of the sample were collected using a Karl Fischer (KF) moisture titrator, Metrohm 870/803 from Metrohm, Switzerland. The measurement was performed using the volumetric method, with HYDRANAL Composite-2 (Sigma-Aldrich, P/N: 34806) as the titrant and anhydrous methanol as the solvent. The stirring time was 30 seconds. The specific procedure involves weighing approximately 50 mg of the sample (accurate to 0.001 g), testing the moisture content using the KF moisture titrator, performing two parallel determinations, and taking the average value.

Liquid-state nuclear magnetic resonance (1H NMR) in the present disclosure: the sample analysis was performed using a Bruker 400M NMR spectrometer, with DMSO-d6 as the solvent.

Solid-state nuclear magnetic resonance (ssNMR) in the present disclosure: the data were collected using a Bruker 500 MHz wide-bore solid-state nuclear magnetic resonance spectrometer with a 4 mm dual resonance magic angle spinning probe. The cross-polarization/magic angle spinning (CP/MAS) method was utilized. To avoid the influence of spinning sidebands, sideband suppression techniques (TOSS) were simultaneously integrated to collect the 13C fingerprint spectrum of the sample. The cross-polarization matching conditions were optimized using adamantane, and the chemical shift was corrected using the high-field signal of adamantane (29.5 ppm). The magic angle spinning speed was 8 kHz, the 1H 90-degree pulse was 3.45 µs, and the decoupling method was TPPM. The specific experimental conditions are as follows:
Experiment 2#: pulse delay: 8 s; contact time: 2 ms; number of accumulations: 1500.
Experiment 3#: pulse delay: 2 s; contact time: 2 ms; number of accumulations: 1500.
Experiment 4#: pulse delay: 2 s; contact time: 0.2 ms; number of accumulations: 3100.

The Chinese meanings of the solvent abbreviations or English used in the present disclosure are shown in Table 19 below:

**Table 19 Solvents in Chinese and English**

| English | Chinese | English | Chinese |
|---|---|---|---|
| MeOH | Methanol | EtOH | Ethanol |
| IPA | Isopropanol | 2-Butanol | sec-Butanol |
| Acetone | Dimethyl ketone | MEK | Methyl ethyl ketone |
| MIBK | Methyl isobutyl ketone | EtOAc | Ethyl acetate |
| IPAc | Isopropyl acetate | MTBE | Methyl *tert*-butyl ether |
| CPME | Cyclopentyl methyl ether | THF | Tetrahydrofuran |
| 1,4-Dioxane | 1,4-Dioxane | DMSO | Dimethyl sulfoxide |
| DMF | Dimethylformamide | DCM | Dichloromethane |
| CHCl₃ | Trichloromethane | *n*-Heptane | *n*-Heptane |
| 2-MeTHF | 2-Methyltetrahydrofuran | Toluene | Toluene |
| H₂O | Water | ACN | Acetonitrile |
| Butyl acetate | n-Butyl acetate | Methyl acetate | MeOAc |
| Cumene | Isopropylbenzene | Anisole | Methoxybenzine |
| Hexane | Hexane | -- | -- |

To enable those skilled in the art to better understand the present disclosure, the following specific examples of the present disclosure are described.

### Example 1: Preparation of Crystal Form A

At room temperature, the compound of formula (I) was stirred in a mixture of anisole/n-heptane (4:3, v/v) to precipitate a solid. The solid was then filtered and dried under vacuum at 45°C for 36 hours to obtain the crystal form A.

### Example 2: Preparation of Crystal Form B

2.0 g of the compound of formula (I) was dissolved in 10 mL of IPAC and heated to 60°C. Heptane (40 mL) was added dropwise, and the mixture was cooled to 40°C, resulting in the precipitation of a large amount of solid. An additional 10 mL of heptane was added, and the mixture was stirred for 2 hours, filtered, and dried under vacuum at 45°C for 36 hours to obtain the crystal form B.

### Example 3: Preparation of Crystal Form D

At room temperature, the compound of formula (I) was stirred in a mixture of MeOH/H₂O (9:2, v/v) to precipitate a solid. The solid was then filtered and dried under vacuum at 45°C for 36 hours to obtain the crystal form D.

### Example 4: Preparation of Crystal Form C

The crystal form D was heated to 137°C to obtain the crystal form C.

### Example 5: Preparation of Crystal Form E

At room temperature, the compound of formula (I) was stirred in a mixture of CPME/Heptane (2:3, v/v) to precipitate a solid. The solid was then filtered and dried under vacuum at 45°C for 36 hours to obtain the crystal form E.

### Example 6: Preparation of Crystal Form F (including the step of salt formation)

0.4 g of the compound of formula (I) was dissolved in 3.0 mL of EtOH at room temperature, with stirring until dissolved. An ethanolic solution of diethylamine (diethylamine: 69.4 mg; ethanol: 0.8 mL) was slowly added thereto, and the mixture was stirred at room temperature for 19 hours to precipitate a solid. The solid was filtered and dried under vacuum at 30°C for 3 hours to obtain the crystal form F.

### Example 7: Crystal Form G

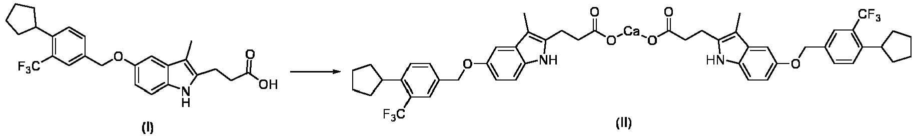

Method 1: The compound of formula (I) (0.2651 kg, 0.595 mol) and anhydrous ethanol (0.9488 kg) were added to a reactor, and the mixture was heated to 35-45°C with stirring for 5-30 minutes. Sodium ethoxide solution (sodium ethoxide: 0.035 kg, 0.514 mol; anhydrous ethanol: 0.6310 kg) was added thereto and stirred for 2-3 hours. Calcium chloride solution (calcium chloride: 0.040 kg, 0.357 mol; purified water: 0.847 kg) was added dropwise thereto and stirred for 1-3 hours. The mixture was cooled to 20-30°C and stirred for 4-6 hours, then filtered. The filter cake was rinsed with purified water and ethanol. The filter cake was dried under reduced pressure at 25-35°C to obtain 0.2185 kg of the compound of formula (II) as the crystal form G, with a yield of 79.1%.

Method 2: 10.72 g of the compound of formula (I) was weighed into a single-neck flask and 105 mL of EtOH was added thereto. The mixture was stirred with a magnetic stirrer for about 0.5 hours, then 1.64 g of C₂H₅Ona was added, and the resulting mixture was stirred at room temperature for 1.5 hours. Anhydrous CaCh (1.5 g) was dissolved in EtOH/H₂O (2:1, v/v, 45 mL), and added to 1% solution of seed crystal (crystal form G) in calcium chloride. The calcium chloride solution was then added dropwise thereto and stirred for 16 hours. The suspension was taken, and the XRPD of the sample was determined, showing as the crystal form D. The suspension was filtered, and the filter cake was washed twice each with H₂O and EtOH. The XRPD of wet sample was determined, showing as the crystal form J. After drying under reduced pressure at 50°C for 4 hours, the XRPD was determined again, showing the crystal form G.

Method 3: 21 mg of the compound of formula (I) was added with 2 mg of Ca(OH)₂ and 0.2 mL of EtOH/H₂O (9:1, v/v). The mixture was stirred with a magnetic stirrer at room temperature for 3 days, then filtered and dried under vacuum at 50°C for 2 hours to obtain the crystal form G.

Method 4: 21 mg of the compound of formula (I) was added with 2 mg of Ca(OH)₂ and 0.2 mL of IPA/H₂O (9:1, v/v). The mixture was stirred with a magnetic stirrer at room temperature for 4 days, then filtered and dried under vacuum at 50°C for 2 hours to obtain the crystal form G.

For the product obtained by filtration in Method 3, different drying times, temperatures, or vacuum levels were adopted and the water and solvent content was respectively measured, and XRPD was detected. The results are shown in Table 20 below.

**Table 20**

| Solvent (Ethanol) Content | Water Content | Analytical Table | Figure |
|---|---|---|---|
| 4.77% | 3.09% | Table 21 | Fig. 18 |
| 3.05% | 3.89% | Table 22 | Fig. 19 |
| 1.97% | 4.37% | Table 23 | Fig. 20 |
| 1.64% | 3.75% | Table 24 | Fig. 21 |

**Analytical Table 21**

| Pos. [°2Th.] | Height [cts] | FWHM Left [°2Th.] | d-spacing [Å] | Rel. Int. [%] |
|---|---|---|---|---|
| 9.02 | 5227 | 0.17 | 9.80 | 100.00 |
| 10.17 | 3060 | 0.18 | 8.69 | 58.50 |
| 13.62 | 949 | 0.22 | 6.49 | 18.20 |
| 14.83 | 374 | 0.23 | 5.97 | 7.20 |
| 15.18 | 1478 | 0.23 | 5.83 | 28.30 |
| 16.48 | 440 | 0.19 | 5.38 | 8.40 |
| 17.01 | 449 | 0.19 | 5.21 | 8.60 |
| 18.51 | 1032 | 0.17 | 4.79 | 19.70 |
| 19.10 | 307 | 0.26 | 4.64 | 5.90 |
| 20.46 | 272 | 0.25 | 4.34 | 5.20 |
| 21.27 | 251 | 0.28 | 4.17 | 4.80 |
| 22.63 | 319 | 0.26 | 3.93 | 6.10 |
| 23.26 | 221 | 0.16 | 3.82 | 4.20 |
| 24.95 | 913 | 0.18 | 3.57 | 17.50 |
| 26.41 | 223 | 0.21 | 3.37 | 4.30 |
| 27.35 | 333 | 0.17 | 3.26 | 6.40 |
| 30.82 | 64.2 | 0.36 | 2.90 | 1.20 |

**Analytical Table 22**

| Pos. [°2Th.] | Height [cts] | FWHM Left [°2Th.] | d-spacing [Å] | Rel. Int. [%] |
|---|---|---|---|---|
| 9.04 | 5672 | 0.18 | 9.78 | 100.00 |
| 10.17 | 2803 | 0.20 | 8.69 | 49.40 |
| 13.60 | 872 | 0.29 | 6.50 | 15.40 |
| 14.83 | 425 | 0.16 | 5.97 | 7.50 |
| 15.19 | 1551 | 0.24 | 5.83 | 27.30 |
| 16.50 | 517 | 0.18 | 5.37 | 9.10 |
| 17.03 | 503 | 0.21 | 5.20 | 8.90 |
| 18.52 | 1135 | 0.19 | 4.79 | 20.00 |
| 19.13 | 320 | 0.22 | 4.64 | 5.60 |
| 20.47 | 260 | 0.27 | 4.34 | 4.60 |
| 21.24 | 276 | 0.29 | 4.18 | 4.90 |
| 22.60 | 369 | 0.28 | 3.93 | 6.50 |
| 23.23 | 222 | 0.43 | 3.83 | 3.90 |
| 24.96 | 1106 | 0.19 | 3.56 | 19.50 |
| 26.41 | 294 | 0.19 | 3.37 | 5.20 |
| 27.35 | 363 | 0.18 | 3.26 | 6.40 |
| 30.80 | 67.1 | 0.39 | 2.90 | 1.20 |

**Analytical Table 23**

| Pos. [°2Th.] | Height [cts] | FWHM Left [°2Th.] | d-spacing [Å] | Rel. Int. [%] |
|---|---|---|---|---|
| 9.00 | 4460 | 0.22 | 9.82 | 100.00 |
| 10.11 | 1992 | 0.23 | 8.74 | 44.70 |
| 13.57 | 732 | 0.25 | 6.52 | 16.40 |
| 14.76 | 462 | 0.16 | 6.00 | 10.40 |
| 15.15 | 1576 | 0.25 | 5.84 | 35.30 |
| 16.43 | 522 | 0.22 | 5.39 | 11.70 |
| 16.97 | 512 | 0.26 | 5.22 | 11.50 |
| 18.47 | 1244 | 0.22 | 4.80 | 27.90 |
| 19.03 | 364 | 0.28 | 4.66 | 8.20 |
| 20.38 | 293 | 0.26 | 4.35 | 6.60 |
| 21.18 | 307 | 0.27 | 4.19 | 6.90 |
| 22.58 | 573 | 0.25 | 3.93 | 12.80 |
| 23.21 | 313 | 0.47 | 3.83 | 7.00 |
| 24.92 | 1092 | 0.25 | 3.57 | 24.50 |
| 26.33 | 368 | 0.18 | 3.38 | 8.30 |
| 27.28 | 436 | 0.23 | 3.27 | 9.80 |
| 30.76 | 128 | 0.26 | 2.90 | 2.90 |

**Analytical Table 24**

| Pos. [°2Th.] | Height [cts] | FWHM Left [°2Th.] | d-spacing [Å] | Rel. Int. [%] |
|---|---|---|---|---|
| 9.01 | 5007 | 0.19 | 9.80 | 100.00 |
| 10.11 | 2643 | 0.19 | 8.74 | 52.80 |
| 13.59 | 884 | 0.21 | 6.51 | 17.70 |
| 14.79 | 427 | 0.20 | 5.98 | 8.50 |
| 15.18 | 1628 | 0.23 | 5.83 | 32.50 |
| 16.45 | 512 | 0.18 | 5.38 | 10.20 |
| 17.01 | 525 | 0.20 | 5.21 | 10.50 |
| 18.50 | 1178 | 0.19 | 4.79 | 23.50 |
| 19.07 | 368 | 0.23 | 4.65 | 7.30 |
| 20.41 | 289 | 0.24 | 4.35 | 5.80 |
| 21.25 | 325 | 0.26 | 4.18 | 6.50 |
| 22.61 | 471 | 0.24 | 3.93 | 9.40 |
| 23.25 | 260 | 0.48 | 3.82 | 5.20 |
| 24.95 | 925 | 0.21 | 3.57 | 18.50 |
| 26.37 | 302 | 0.18 | 3.38 | 6.00 |
| 27.34 | 373 | 0.18 | 3.26 | 7.50 |
| 30.79 | 116 | 0.25 | 2.90 | 2.30 |

Stability verification of crystal form G in different solvents:
Method 1: The crystal form G (50 mg) was added to 0.5 mL of the corresponding solvent (the solvent may be EtOH, H₂O, or a mixture of EtOH/H₂O in the ratios of 970/30, 927/73, 855/145, 704/296, v/v). The mixture was stirred with a magnetic stirrer at room temperature for 4 days, filtered, dried under vacuum at 50°C for 2 hours to obtain the crystal form G.
Method 2: The crystal form G (15 mg) was placed in a 4 mL glass vial, which was then placed inside a 20 mL glass vial containing 3 mL of a volatile solvent (which may be MeOH or EtOH). The 20 mL glass vial was tightly capped and left to stand at room temperature for approximately one week. The sample was then filtered and dried under vacuum at 50°C for 2 hours to obtain the crystal form G.

### Example 8: Preparation of Crystal Form H

The crystal form G (50 mg) was added to 0.5 mL of MeOH. The mixture was stirred with a magnetic stirrer at room temperature for 4 days, then filtered to obtain the crystal form H.

### Example 9: Preparation of Crystal Form I

The crystal form G (50 mg) was added to an HPLC, and 0.5 mL of MeOH was added thereto. The mixture was stirred with a magnetic stirrer at 50°C for 7 days to obtain a suspension. The suspension was then filtered to obtain the crystal form I.

### Example 10: Preparation of Crystal Form J

Method 1: The crystal form G (50 mg) was added to 0.5 mL of solvent (the solvent may be 2-butanol or toluene). The mixture was stirred with a magnetic stirrer at room temperature for 4 days, then filtered to obtain the crystal form J.

Method 2: The crystal form G (50 mg) was added to an HPLC vial, and 0.5 mL of IPA was added thereto. The mixture was stirred with a magnetic stirrer at 50°C for 7 days to obtain a suspension. The suspension was then filtered to obtain the crystal form J.

Method 3: The crystal form G (50 mg) was added to an HPLC vial, and 0.5 mL of a mixture of EtOAc/n-Heptane (1/2, v/v) as added thereto. The mixture was stirred with a magnetic stirrer at 50°C for 7 days to obtain a suspension. The suspension was then filtered to obtain the crystal form J.

Method 4: Approximately 21 mg of the compound of formula (I) from Example 1 (purity of 93 area%) and approximately 2 mg of Ca(OH)₂ were weighed into an HPLC vial; 0.2 mL of a mixture of EtOH/H₂O (9: 1, v/v) was added thereto, and the mixture was stirred with a magnetic stirrer at room temperature. After stirring for 3 days, the mixture was centrifuged, and the XRPD of wet sample was tested, which was the crystal form J.

### Example 11: Preparation of Crystal Form K

The crystal form G (50 mg) was added to an HPLC vial, and 0.5 mL of a mixture of 2-MeTHF/n-Heptane (1/2, v/v) was added thereto. The mixture was stirred with a magnetic stirrer at 50°C for 7 days to obtain a suspension. The suspension was then filtered to obtain the crystal form K.

### Example 12: Preparation of Crystal Form L

Under a nitrogen atmosphere, the crystal form K was heated to 80°C and then cooled to room temperature to obtain the crystal form L.

### Example 13: Preparation of Crystal Form M

The crystal form G (15 mg) was added to a 20 mL glass vial, DMF was added, and the mixture was stirred at room temperature until dissolved. H₂O was gradually added to the vial until a solid appeared. The mixture was then filtered to obtain the crystal form M.

### Example 14: Preparation of Crystal Form N

The crystal form G (15 mg) was added to a 4 mL glass vial, and DMF was added to dissolve it clearly. If dissolved clarification was not achieved, the suspension was subjected to membrane filtration (nylon membrane, pore size 0.22 µm). The resulting solution was respectively added into a 20 mL glass vial containing 4 mL of H₂O. The 20 mL glass vial was tightly capped and left at room temperature until a solid precipitated. The mixture was then filtered to obtain the crystal form N.

### Example 15: Preparation of Crystal Form O

The crystal form G was dissolved in acetone and left to stand for approximately 10 minutes until a solid precipitated. The solid was then filtered to obtain the crystal form O.

### Example 16: Preparation of Crystal Form P

The crystal form G (15 mg) was added to a 20 mL glass vial, acetone was added thereto, and the mixture was stirred at room temperature until dissolved. MTBE was gradually added to the vial until a solid appeared. The mixture was then filtered to obtain the crystal form P.

### Example 17: Preparation of Crystal Form Q

110 mg of the compound of formula (I) was added to a 20 mL single-neck flask, and 1 mL of EtOH was added. The mixture was stirred with a magnetic stirrer until clear, followed by the addition of sodium ethoxide (16 mg). The mixture was stirred with a magnetic stirrer at 40°C for 1 hour, and then 0.1 mL of a strontium chloride aqueous solution (300 mg/mL) was added dropwise thereto. The reaction mixture was stirred and reacted at 28°C for 12 hours, filtered, and dried to obtain the crystal form Q.

### Example 18

The inventors found that some of the disclosed crystal forms A to Q had poor reproducibility in preparation or could not be purified. Consequently, the inventors selected four crystal forms with good reproducibility and ease of purification: crystal forms A, B, G, and Q. These crystal forms were subjected to stability tests under identical conditions of high temperature, high humidity, and light exposure. The test protocols and results are shown in Table 25, and the changes in shape are shown in Table 26.

**Table 25**

| Condition | Crystal form A | Crystal form B | Crystal form G | Crystal form Q |
|---|---|---|---|---|
| Initial purity | 99.28% | 99.44% | 99.60% | 96.52% |
| After 30 days at high temperature (60°C) | 94.57% | 97.21% | 99.12% | 95.32% |
| After 30 days at a temperature of 25°C with a relative humidity of 90% ± 5% | 98.49% | 98.50% | 98.78% | 95.26% |
| After 30 days at a light intensity of 4500 Lx ± 500 Lx | 94.45% | 94.55% | 96.02% | 93.15% |

**Table 26**

| Condition | | Crystal form A | Crystal form B | Crystal form G | Crystal form Q |
|---|---|---|---|---|---|
| Initial State | | Off-white solid | Off-white solid | Off-white solid | Off-white solid |
| High Temperature (60°C) | 5 Days | Off-white solid | Off-white solid | Off-white solid | Off-white solid |
| | 10 Days | Pale yellow solid | Off-white solid | Off-white solid | Off-white solid |
| | 30 Days | Yellow solid | Off-white solid | Off-white solid | Off-white solid |
| Temperature of 25°C, relative humidity of 90% ± 5% | 5 Days | Off-white solid | Off-white solid | Off-white solid | Off-white solid |
| | 10 Days | Off-white solid | Off-white solid | Off-white solid | Off-white solid |
| | 30 Days | Off-white solid | Off-white solid | Off-white solid | Off-white solid |
| Light intensity of 4500 Lx ± 500 Lx | 5 Days | Off-white solid | Off-white solid | Off-white solid | Off-white solid |
| | 10 Days | Pale yellow solid | Pale yellow solid | Pale yellow solid | Pale yellow solid |
| | 30 Days | Yellow solid | Yellow solid | Yellow solid | Yellow solid |

It can be seen from Tables 25 and 26 above that the crystal form G exhibits higher stability under high temperature, high humidity, and light exposure conditions compared to the other crystal forms.

### Example 19

The purity of crystal forms A, B, G, and Q, under identical mass and packaging conditions, was investigated at storage conditions of 25°C and 40°C, as shown in Table 27.

**Table 27**

| Days | Crystal form A | | Crystal form B | | Crystal form G | | Crystal form Q | |
|---|---|---|---|---|---|---|---|---|
| | 25°C | 40°C | 25°C | 40°C | 25°C | 40°C | 25°C | 40°C |
| 0 Days | 99.03% | 99.03% | 99.14% | 99.14% | 99.48% | 99.48% | 96.21% | 96.21% |
| 15 Days | 98.91% | 98.73% | 99.05% | 98.88% | 99.42% | 99.28% | 96.24% | 96.07% |
| 30 Days | 98.93% | 98.42% | 99.00% | 98.73% | 99.43% | 99.27% | 96.31% | 95.99% |
| 45 Days | 98.87% | 98.30% | 98.93% | 98.68% | 99.43% | 99.24% | 96.29% | 95.90% |
| 60 Days | 98.85% | 98.15% | 98.94% | 98.66% | 99.41% | 99.16% | 96.25% | 95.76% |
| 90 Days | 98.73% | 97.69% | 98.86% | 98.40% | 99.36% | 99.01% | 96.18% | 95.61% |

As can be seen from Table 27 above, the crystal form G exhibits higher stability when stored at 25°C and 40°C for 90 days.

## Claims

1. A calcium salt of a compound of formula (I),

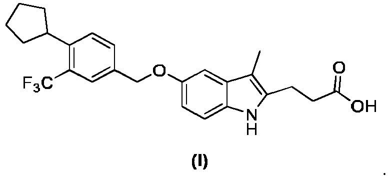

2. The calcium salt according to claim 1, which is

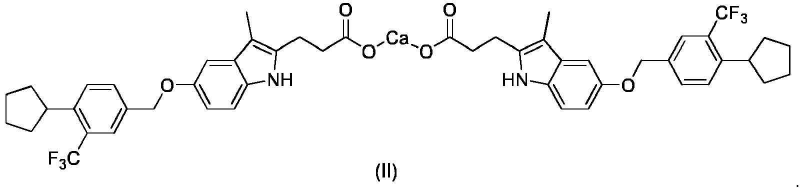

3. A crystal form G of a compound of formula (II), wherein the crystal form G has an X-ray powder diffraction pattern comprising characteristic peaks at the following 2θ angles: 8.98 ± 0.2°, 10.12 ± 0.2°, 13.56 ± 0.2°, 14.66 ± 0.2°, 15.22 ± 0.2°, 18.48 ± 0.2°.

4. The crystal form G according to claim 3, wherein the X-ray powder diffraction pattern of the crystal form G comprises characteristic peaks at the following 2Θ angles: 8.98 ± 0.2°, 10.12 ± 0.2°, 13.56 ± 0.2°, 14.66 ± 0.2°, 15.22 ± 0.2°, 16.34 ± 0.2°, 16.92 ± 0.2°, 18.48 ± 0.2°, 22.67 ± 0.2°, 23.06 ± 0.2°.

5. The crystal form G according to claim 3 or 4, wherein the X-ray powder diffraction pattern of the crystal form G is an X-ray powder diffraction pattern basically as shown in Fig. 7.

6. The crystal form G according to claim 3 or 4, wherein the crystal form G further comprises water and a solvent;
wherein
the solvent is selected from ethanol and isopropanol;
the content of the solvent ranges from 0.1% to 6.0%;
the content of water ranges from 0.1% to 4.0%.

7. A method for preparing a crystal form G, comprising: dissolving a compound of formula (I) in a sodium ethoxide solution, followed by stirring in the presence of calcium chloride, filtering, and drying under reduced pressure to obtain the crystal form G.

8. A pharmaceutical composition, comprising the calcium salt according to claim 1 or 2, or the crystal form G according to any one of claims 3 to 6.

9. A use of the calcium salt according to claim 1 or 2, the crystal form G according to any one of claims 3 to 6, or the pharmaceutical composition according to claim 8 in the manufacture of a medicament for an S1P1 receptor-related disease.

10. The use according to claim 9, wherein the S1P1 receptor-related disease is selected from ulcerative colitis, Crohn's disease, multiple sclerosis, systemic lupus erythematosus, lupus nephritis, rheumatoid arthritis, primary biliary cholangitis, atopic dermatitis, intracerebral hemorrhage, graft-versus-host disease, psoriasis, type I diabetes, acne, microbial infections or microbial diseases, and viral infections or viral diseases.
